# EUROPEAN PATENT APPLICATION

(11) **EP 3 572 426 A1**
(43) Date of publication of application: **27.11.2019**
(21) Application number: 18741009.7
(22) Date of filing: 22.01.2018
(51) Int. Cl.: C07K 16/28, C07K 16/46, C12P 21/08, G01N 33/53, G01N 33/574, C12N 15/09

(54) **ANTI-EPHA2 ANTIBODY AND IMMUNOLOGICAL DETECTION OF EPHA2 USING SAME**

(30) Priority: 23.01.2017 JP 2017009349
(71) Applicant: Toyo University, Tokyo 112-8606 (JP)
(72) Inventor: KATO, Kazunori, Kawagoe-shi Saitama 350-8585 (JP); AGATSUMA, Toshinori, Tokyo 103-8426 (JP); HASEGAWA, Jun, Tokyo 103-8426 (JP)
(74) Representative: D Young & Co LLP
(86) International application number: PCT/JP2018/001730
(87) International publication number: WO 2018/135653

(57) **Abstract**

A monoclonal antibody specifically binding to EPHA2, comprising a heavy chain variable region consisting of the amino acid sequence as set forth in SEQ ID NO: 20 and a light chain variable region consisting of the amino acid sequence as set forth in SEQ ID NO: 22, or a functional fragment of the monoclonal antibody specifically binding to EPHA2, and a monoclonal antibody specifically binding to EPHA2, comprising a heavy chain variable region consisting of the amino acid sequence as set forth in SEQ ID NO: 15 and a light chain variable region consisting of the amino acid sequence as set forth in SEQ ID NO: 18, or a functional fragment of the monoclonal antibody specifically binding to EPHA2.

SEQUENCE LISTING

<110>  Toyo University

<120>  An anti EPHA2 antibody and an immunological detection of EPHA2
       using the antibody

<130>  PH-7210-PCT

<150>  JP 2017-009349
<151>  2017-01-23

<160>  34

<170>  PatentIn version 3.5

<210>  1
<211>  55
<212>  DNA
<213>  Artificial

<220>
<223>  Primer

<400>  1
ggggacaagt ttgtacaaaa aagcaggctt cggggatcgg accgagagcg agaag          55


<210>  2
<211>  66
<212>  DNA
<213>  Artificial

<220>
<223>  Primer

<400>  2
ggggaccact ttgtacaaga aagctgggtc ctagatgggg atccccacag tgttcacctg     60

gtcctt                                                                66


<210>  3
<211>  29
<212>  DNA
<213>  Artificial

<220>
<223>  Primer

<400>  3
ctgtggggat ccccatcgac ccagctttc                                       29


<210>  4
<211>  30
<212>  DNA
<213>  Artificial

<220>
<223>  Primer

<400>  4

gatggggatc cccacagtgt tcacctggtc                                    30

<210>  5
<211>  2961
<212>  DNA
<213>  Homo sapiens

<400>  5
tcggggatcg gaccgagagc gagaagcgcg gcatggagct ccaggcagcc cgcgcctgct      60

tcgccctgct gtggggctgt gcgctggccg cggccgcggc ggcgcagggc aaggaagtgg     120

tactgctgga ctttgctgca gctggagggg agctcggctg gctcacacac ccgtatggca     180

aagggtggga cctgatgcag aacatcatga atgacatgcc gatctacatg tactccgtgt     240

gcaacgtgat gtctggcgac caggacaact ggctccgcac caactgggtg taccgaggag     300

aggctgagcg tatcttcatt gagctcaagt ttactgtacg tgactgcaac agcttccctg     360

gtggcgccag ctcctgcaag gagactttca acctctacta tgccgagtcg gacctggact     420

acggcaccaa cttccagaag cgcctgttca ccaagattga caccattgcg cccgatgaga     480

tcaccgtcag cagcgacttc gaggcacgcc acgtgaagct gaacgtggag gagcgctccg     540

tggggccgct cacccgcaaa ggcttctacc tggccttcca ggatatcggt gcctgtgtgg     600

cactactctc cgtccgtgtc tactacaaga agtgccccga gctgctgcag ggcctggccc     660

acttccctga gaccatcgcc ggctctgatg caccttccct ggccactgtg gccggcacct     720

gtgtggacca tgccgtggtg ccaccggggg gtgaagagcc ccgtatgcac tgtgcagtgg     780

atggcgagtg gctggtgccc attgggcagt gcctgtgcca ggcaggctac gagaaggtgg     840

aggatgcctg ccaggcctgc tcgcctggat tttttaagtt tgaggcatct gagagcccct     900

gcttggagtg ccctgagcac acgctgccat cccctgaggg tgccacctcc tgcgagtgtg     960

aggaaggctt cttccgggca cctcaggacc cagcgtcgat gccttgcaca cgaccccctt    1020

ccgccccaca ctacctcaca gccgtgggca tgggtgccaa ggtggagctg cgctggacgc    1080

cccctcagga cagcgggggc cgcgaggaca ttgtctacag cgtcacctgc gaacagtgct    1140

ggcccgagtc tggggaatgc gggccgtgtg aggccagtgt gcgctactcg gagcctcctc    1200

acggactgac ccgcaccagt gtgacagtga gcgacctgga gccccacatg aactacacct    1260

tcaccgtgga ggcccgcaat ggcgtctcag gcctggtaac cagccgcagc ttccgtactg    1320

ccagtgtcag catcaaccag acagagcccc ccaaggtgag gctggagggc cgcagcacca    1380

cctcgcttag cgtctcctgg agcatccccc cgccgcagca gagccgagtg tggaagtacg    1440

aggtcactta ccgcaagaag ggagactcca acagctacaa tgtgcgccgc accgagggtt    1500

tctccgtgac cctggacgac ctggccccag acaccaccta cctggtccag gtgcaggcac    1560

tgacgcagga gggccagggg gccggcagca aggtgcacga attccagacg ctgtccccgg    1620

agggatctgg caacttggcg gtgattggcg gcgtggctgt cggtgtggtc ctgcttctgg 1680

tgctggcagg agttggcttc tttatccacc gcaggaggaa gaaccagcgt gcccgccagt 1740

ccccggagga cgtttacttc tccaagtcag aacaactgaa gcccctgaag acatacgtgg 1800

acccccacac atatgaggac cccaaccagg ctgtgttgaa gttcactacc gagatccatc 1860

catcctgtgt cactcggcag aaggtgatcg gagcaggaga gtttggggag gtgtacaagg 1920

gcatgctgaa gacatcctcg gggaagaagg aggtgccggt ggccatcaag acgctgaaag 1980

ccggctacac agagaagcag cgagtggact tcctcggcga ggccggcatc atgggccagt 2040

tcagccacca caacatcatc cgcctagagg gcgtcatctc caaatacaag cccatgatga 2100

tcatcactga gtacatggag aatggggccc tggacaagtt ccttcgggag aaggatggcg 2160

agttcagcgt gctgcagctg gtgggcatgc tgcggggcat cgcagctggc atgaagtacc 2220

tggccaacat gaactatgtg caccgtgacc tggctgcccg caacatcctc gtcaacagca 2280

acctggtctg caaggtgtct gactttggcc tgtcccgcgt gctggaggac gaccccgagg 2340

ccacctacac caccagtggc ggcaagatcc ccatccgctg gaccgccccg gaggccattt 2400

cctaccggaa gttcacctct gccagcgacg tgtggagctt tggcattgtc atgtgggagg 2460

tgatgaccta tggcgagcgg ccctactggg agttgtccaa ccacgaggtg atgaaagcca 2520

tcaatgatgg cttccggctc cccacaccca tggactgccc ctccgccatc taccagctca 2580

tgatgcagtg ctggcagcag gagcgtgccc gccgccccaa gttcgctgac atcgtcagca 2640

tcctggacaa gctcattcgt gcccctgact ccctcaagac cctggctgac tttgaccccc 2700

gcgtgtctat ccggctcccc agcacgagcg gctcggaggg ggtgcccttc cgcacggtgt 2760

ccgagtggct ggagtccatc aagatgcagc agtatacgga gcacttcatg gcggccggct 2820

acactgccat cgagaaggtg gtgcagatga ccaacgacga catcaagagg attggggtgc 2880

ggctgcccgg ccaccagaag cgcatcgcct acagcctgct gggactcaag gaccaggtga 2940

acactgtggg gatccccatc g 2961

<210> 6
<211> 976
<212> PRT
<213> Homo sapiens

<400> 6

<210>  7
<211>  30
<212>  DNA
<213>  Artificial

<220>
<223>  Primer

<400>  7
aaaaagctta tggagctcca ggcagcccgc                                        30

<210>  8
<211>  30
<212>  DNA
<213>  Artificial

<220>
<223>  Primer

<400>  8
aaagggccct cagttgccag atccctccgg                                        30


<210>  9
<211>  22
<212>  DNA
<213>  Artificial

<220>
<223>  Primer

<400>  9
caccatggag ctggcggcct tg                                                22


<210>  10
<211>  21
<212>  DNA
<213>  Artificial

<220>
<223>  Primer

<400>  10
tcccactggc acgtccagac c                                                 21


<210>  11
<211>  3804
<212>  DNA
<213>  Homo sapiens

<400>  11
ccgcggccgc ccccttcacc atggagctgg cggccttgtg ccgctggggg ctcctcctcg        60

ccctcttgcc ccccggagcc gcgagcaccc aagtgtgcac cggcacagac atgaagctgc      120

ggctccctgc cagtcccgag acccacctgg acatgctccg ccacctctac cagggctgcc      180

aggtggtgca gggaaacctg gaactcacct acctgcccac caatgccagc ctgtccttcc      240

tgcaggatat ccaggaggtg cagggctacg tgctcatcgc tcacaaccaa gtgaggcagg      300

tcccactgca gaggctgcgg attgtgcgag gcacccagct ctttgaggac aactatgccc      360

tggccgtgct agacaatgga gacccgctga acaataccac ccctgtcaca ggggcctccc      420

caggaggcct gcgggagctg cagcttcgaa gcctcacaga gatcttgaaa ggaggggtct      480

tgatccagcg gaacccccag ctctgctacc aggacacgat tttgtggaag gacatcttcc      540

acaagaacaa ccagctggct ctcacactga tagacaccaa ccgctctcgg gcctgccacc      600

cctgttctcc gatgtgtaag ggctcccgct gctggggaga gagttctgag gattgtcaga      660

gcctgacgcg cactgtctgt gccggtggct gtgcccgctg caaggggcca ctgcccactg      720

actgctgcca tgagcagtgt gctgccggct gcacgggccc caagcactct gactgcctgg      780

cctgcctcca cttcaaccac agtggcatct gtgagctgca ctgcccagcc ctggtcacct 840
acaacacaga cacgtttgag tccatgccca atcccgaggg ccggtataca ttcggcgcca 900
gctgtgtgac tgcctgtccc tacaactacc tttctacgga cgtgggatcc tgcaccctcg 960
tctgccccct gcacaaccaa gaggtgacag cagaggatgg aacacagcgg tgtgagaagt 1020
gcagcaagcc ctgtgcccga gtgtgctatg gtctgggcat ggagcacttg cgagaggtga 1080
gggcagttac cagtgccaat atccaggagt ttgctggctg caagaagatc tttgggagcc 1140
tggcatttct gccggagagc tttgatgggg acccagcctc caacactgcc ccgctccagc 1200
cagagcagct ccaagtgttt gagactctgg aagagatcac aggttaccta tacatctcag 1260
catggccgga cagcctgcct gacctcagcg tcttccagaa cctgcaagta atccggggac 1320
gaattctgca caatggcgcc tactcgctga ccctgcaagg gctgggcatc agctggctgg 1380
ggctgcgctc actgagggaa ctgggcagtg gactggccct catccaccat aacacccacc 1440
tctgcttcgt gcacacggtg ccctgggacc agctctttcg gaacccgcac caagctctgc 1500
tccacactgc caaccggcca gaggacgagt gtgtgggcga gggcctggcc tgccaccagc 1560
tgtgcgcccg agggcactgc tggggtccag ggcccaccca gtgtgtcaac tgcagccagt 1620
tccttcgggg ccaggagtgc gtggaggaat gccgagtact gcaggggctc cccagggagt 1680
atgtgaatgc caggcactgt ttgccgtgcc accctgagtg tcagccccag aatggctcag 1740
tgacctgttt tggaccggag gctgaccagt gtgtggcctg tgcccactat aaggaccctc 1800
ccttctgcgt ggcccgctgc cccagcggtg tgaaacctga cctctcctac atgcccatct 1860
ggaagtttcc agatgaggag ggcgcatgcc agccttgccc catcaactgc acccactcct 1920
gtgtggacct ggatgacaag ggctgccccg ccgagcagag agccagccct ctgacgtcca 1980
tcatctctgc ggtggttggc attctgctgg tcgtggtctt gggggtggtc tttgggatcc 2040
tcatcaagcg acggcagcag aagatccgga agtacacgat gcggagactg ctgcaggaaa 2100
cggagctggt ggagccgctg acacctagcg gagcgatgcc caaccaggcg cagatgcgga 2160
tcctgaaaga gacggagctg aggaaggtga aggtgcttgg atctggcgct tttggcacag 2220
tctacaaggg catctggatc cctgatgggg agaatgtgaa aattccagtg gccatcaaag 2280
tgttgaggga aaacacatcc cccaaagcca acaaagaaat cttagacgaa gcatacgtga 2340
tggctggtgt gggctcccca tatgtctccc gccttctggg catctgcctg acatccacgg 2400
tgcagctggt gacacagctt atgccctatg gctgcctctt agaccatgtc cgggaaaacc 2460
gcggacgcct gggctcccag gacctgctga actggtgtat gcagattgcc aaggggatga 2520
gctacctgga ggatgtgcgg ctcgtacaca gggacttggc cgctcggaac gtgctggtca 2580
agagtcccaa ccatgtcaaa attacagact tcgggctggc tcggctgctg gacattgacg 2640
agacagagta ccatgcagat gggggcaagg tgcccatcaa gtggatggcg ctggagtcca 2700

ttctccgccg gcggttcacc caccagagtg atgtgtggag ttatggtgtg actgtgtggg 2760

agctgatgac ttttgggccc aaaccttacg atgggatccc agcccgggag atccctgacc 2820

tgctggaaaa gggggagcgg ctgccccagc cccccatctg caccattgat gtctacatga 2880

tcatggtcaa atgttggatg attgactctg aatgtcggcc aagattccgg gagttggtgt 2940

ctgaattctc ccgcatggcc agggaccccc agcgctttgt ggtcatccag aatgaggact 3000

tgggcccagc cagtcccttg gacagcacct tctaccgctc actgctggag gacgatgaca 3060

tgggggacct ggtggatgct gaggagtatc tggtacccca gcagggcttc ttctgtccag 3120

accctgcccc gggcgctggg ggcatggtcc accacaggca ccgcagctca tctaccagga 3180

gtggcggtgg ggacctgaca ctagggctgg agccctctga agaggaggcc cccaggtctc 3240

cactggcacc ctccgaaggg gctggctccg atgtatttga tggtgacctg ggaatggggg 3300

cagccaaggg gctgcaaagc ctccccacac atgaccccag ccctctacag cggtacagtg 3360

aggaccccac agtacccctg ccctctgaga ctgatggcta cgttgccccc ctgacctgca 3420

gcccccagcc tgaatatgtg aaccagccag atgttcggcc ccagccccct tcgccccgag 3480

agggccctct gcctgctgcc cgacctgctg gtgccactct ggaaagggcc aagactctct 3540

ccccagggaa gaatggggtc gtcaaagacg tttttgcctt tgggggtgcc gtggagaacc 3600

ccgagtactt gacaccccag ggaggagctg cccctcagcc ccaccctcct cctgccttca 3660

gcccagcctt cgacaacctc tattactggg accaggaccc accagagcgg ggggctccac 3720

ccagcacctt caaagggaca cctacggcag agaacccaga gtacctgggt ctggacgtgc 3780

cagtgggaaa gggtgggcgc gccg 3804

<210> 12
<211> 30
<212> DNA
<213> Artificial

<220>
<223> Primer

<400> 12
catcccaggg tcaccatgga gttagtttgg 30

<210> 13
<211> 23
<212> DNA
<213> Artificial

<220>
<223> Primer

<400> 13
aagcagtggt atcaacgcag agt 23

<210>  14
<211>  348
<212>  DNA
<213>  Mus musculus

<400>  14
cagatccagt tggtgcagtc tggacctgag ctgaagaagc ctggagagac agtcaagatc        60

tcctgcaagg gttctgggta taccttcaca aactatggaa tgaactgggt gaagcaggct       120

ccaggaaagg gtttaaagtg gatggcctgg ataaacaccg acactggaga gccaatattt       180

gttgaagagt tcaagggacg gtttgccttc tctttggaaa cctctgccag cactgcctat       240

ttgcagatca acaacctcac aaatgaggac acggctacat atttctgtgc aagatctatg       300

gggggctttg ctaactgggg ccaagggact ctggtcactg tctctgca                    348


<210>  15
<211>  116
<212>  PRT
<213>  Mus musculus

<400>  15

Gln Ile Gln Leu Val Gln Ser Gly Pro Glu Leu Lys Lys Pro Gly Glu
1               5                   10                  15


Thr Val Lys Ile Ser Cys Lys Gly Ser Gly Tyr Thr Phe Thr Asn Tyr
            20                  25                  30


Gly Met Asn Trp Val Lys Gln Ala Pro Gly Lys Gly Leu Lys Trp Met
        35                  40                  45


Ala Trp Ile Asn Thr Asp Thr Gly Glu Pro Ile Phe Val Glu Glu Phe
    50                  55                  60


Lys Gly Arg Phe Ala Phe Ser Leu Glu Thr Ser Ala Ser Thr Ala Tyr
65                  70                  75                  80


Leu Gln Ile Asn Asn Leu Thr Asn Glu Asp Thr Ala Thr Tyr Phe Cys
                85                  90                  95


Ala Arg Ser Met Gly Gly Phe Ala Asn Trp Gly Gln Gly Thr Leu Val
            100                 105                 110


Thr Val Ser Ala
        115


<210>  16
<211>  30
<212>  DNA
<213>  Artificial

<220>
<223>  Primer

<400>  16
agtccaactg ttcaggacgc cattttgtcg                                        30


<210>  17
<211>  324
<212>  DNA
<213>  Mus musculus

<400>  17
gacattgtga tgactcagtc tccagccacc ctgtctgtga ctccaggaga tagagtctct       60

ctttcctgca gggccagcca gaatattagc gactacttac actggtatca acaaaaatca      120

catgaatctc caaggcttct catcaaatat gcttcccaat ccatctctgg gatcccctcc      180

aggttcagtg gcagtggatc agggtcagat ttcactctca ctatcaacag tgtggaacct      240

gaagatgttg caatgtatta ctgtcaaaat ggtcacacct ttcccacgtt cggtgctggg      300

accaagctgg agctgaaacg ggct                                             324


<210>  18
<211>  108
<212>  PRT
<213>  Mus musculus

<400>  18

Asp Ile Val Met Thr Gln Ser Pro Ala Thr Leu Ser Val Thr Pro Gly
1               5                   10                  15


Asp Arg Val Ser Leu Ser Cys Arg Ala Ser Gln Asn Ile Ser Asp Tyr
            20                  25                  30


Leu His Trp Tyr Gln Gln Lys Ser His Glu Ser Pro Arg Leu Leu Ile
        35                  40                  45


Lys Tyr Ala Ser Gln Ser Ile Ser Gly Ile Pro Ser Arg Phe Ser Gly
    50                  55                  60


Ser Gly Ser Gly Ser Asp Phe Thr Leu Thr Ile Asn Ser Val Glu Pro
65                  70                  75                  80


Glu Asp Val Ala Met Tyr Tyr Cys Gln Asn Gly His Thr Phe Pro Thr
                85                  90                  95


Phe Gly Ala Gly Thr Lys Leu Glu Leu Lys Arg Ala
            100                 105


<210>  19

<211>  360
<212>  DNA
<213>  Mus musculus

<400>  19
gaagtgcagc tggtggagtc tgggggaggc ttagtgaagc ctggagggtc ccttaaactc      60

tcctgtgaag cctctggatt cactttcagt gactattaca tgtattgggt tcggcagact     120

ccggaaaaga ggctggagtg ggtcgcaacc attagtgatg atggtagttt cacccactat     180

ctagacagtg tgaaggggcg attcaccatc tccagagaca atgcccagaa caacctgtac     240

ctccaaatga gtagtctgaa gtctgacgac acagccatgt attactgtgc aagagacggc     300

tataggtacg acaggccgtt tgcttactgg ggccaaggga ctctggtcac tgtctctgca     360


<210>  20
<211>  120
<212>  PRT
<213>  Mus musculus

<400>  20

Glu Val Gln Leu Val Glu Ser Gly Gly Gly Leu Val Lys Pro Gly Gly
1               5                   10                  15


Ser Leu Lys Leu Ser Cys Glu Ala Ser Gly Phe Thr Phe Ser Asp Tyr
            20                  25                  30


Tyr Met Tyr Trp Val Arg Gln Thr Pro Glu Lys Arg Leu Glu Trp Val
        35                  40                  45


Ala Thr Ile Ser Asp Asp Gly Ser Phe Thr His Tyr Leu Asp Ser Val
    50                  55                  60


Lys Gly Arg Phe Thr Ile Ser Arg Asp Asn Ala Gln Asn Asn Leu Tyr
65                  70                  75                  80


Leu Gln Met Ser Ser Leu Lys Ser Asp Asp Thr Ala Met Tyr Tyr Cys
                85                  90                  95


Ala Arg Asp Gly Tyr Arg Tyr Asp Arg Pro Phe Ala Tyr Trp Gly Gln
            100                 105                 110


Gly Thr Leu Val Thr Val Ser Ala
        115                 120


<210>  21
<211>  327
<212>  DNA
<213>  Mus musculus

<400>  21

gacattgtga tgacccagtc tcacaaattc atgtccacat cagtaggaga cagggtccgc      60

atcacctgca aggccagtca ggatgtgaat actgctgtag cctggtatca acaaaaacca      120

gggcaatctc ctaaactact gatttactgg gcatccaccc ggcacactgg agtccctgat      180

cgattcacag gcagtggatc tgggacagat tattctctca ccatcagcag tgtgcaggct      240

gaagacctgg cactttatta ctgtcaacaa cattatagca ctccgtacac gttcggaggg      300

gggaccaagc tggagataaa acgggct                                          327

<210>  22
<211>  109
<212>  PRT
<213>  Mus musculus

<400>  22

Asp Ile Val Met Thr Gln Ser His Lys Phe Met Ser Thr Ser Val Gly
1               5                   10                  15

Asp Arg Val Arg Ile Thr Cys Lys Ala Ser Gln Asp Val Asn Thr Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Asp Arg Phe Thr Gly
    50                  55                  60

Ser Gly Ser Gly Thr Asp Tyr Ser Leu Thr Ile Ser Ser Val Gln Ala
65                  70                  75                  80

Glu Asp Leu Ala Leu Tyr Tyr Cys Gln Gln His Tyr Ser Thr Pro Tyr
                85                  90                  95

Thr Phe Gly Gly Gly Thr Lys Leu Glu Ile Lys Arg Ala
            100                 105

<210>  23
<211>  5
<212>  PRT
<213>  Mus musculus

<400>  23

Asn Tyr Gly Met Asn
1               5

<210>  24
<211>  17
<212>  PRT
<213>  Mus musculus

<400>  24

Trp Ile Asn Thr Asp Thr Gly Glu Pro Ile Phe Val Glu Glu Phe Lys
1               5                   10                  15

Gly

<210>  25
<211>  7
<212>  PRT
<213>  Mus musculus

<400>  25

Ser Met Gly Gly Phe Ala Asn
1               5

<210>  26
<211>  11
<212>  PRT
<213>  Mus musculus

<400>  26

Arg Ala Ser Gln Asn Ile Ser Asp Tyr Leu His
1               5                   10

<210>  27
<211>  7
<212>  PRT
<213>  Mus musculus

<400>  27

Tyr Ala Ser Gln Ser Ile Ser
1               5

<210>  28
<211>  8
<212>  PRT
<213>  Mus musculus

<400>  28

Gln Asn Gly His Thr Phe Pro Thr
1               5

<210>  29
<211>  5
<212>  PRT
<213>  Mus musculus

<400>  29

Asp Tyr Tyr Met Tyr

1                   5


<210>  30
<211>  17
<212>  PRT
<213>  Mus musculus

<400>  30

Thr Ile Ser Asp Asp Gly Ser Phe Thr His Tyr Leu Asp Ser Val Lys
1               5                   10                  15


Gly




<210>  31
<211>  11
<212>  PRT
<213>  Mus musculus

<400>  31

Asp Gly Tyr Arg Tyr Asp Arg Pro Phe Ala Tyr
1               5                   10


<210>  32
<211>  11
<212>  PRT
<213>  Mus musculus

<400>  32

Lys Ala Ser Gln Asp Val Asn Thr Ala Val Ala
1               5                   10


<210>  33
<211>  7
<212>  PRT
<213>  Mus musculus

<400>  33

Trp Ala Ser Thr Arg His Thr
1               5


<210>  34
<211>  9
<212>  PRT
<213>  Mus musculus

<400>  34

Gln Gln His Tyr Ser Thr Pro Tyr Thr
1               5

## Description

### Technical Field

The present invention relates to a novel antibody, a functional fragment of the antibody, a modified form of the antibody, a nucleotide comprising a nucleotide sequence encoding the amino acid sequence of the antibody, a vector into which the nucleotide is inserted, a cell into which the nucleotide or the vector is introduced, a method for producing the antibody, comprising a step of culturing the cell, a pharmaceutical composition, a composition for diagnosis or examination, and the like.

### Background Art

EPHA2 is a receptor-type tyrosine kinase with a molecular weight of 130 kDa having a single-pass transmembrane structure (Non Patent Literature 1). A ligand binding domain and two fibronectin type 3 domains are present in the extracellular region on the N-terminal side of EPHA2, whereas a tyrosine kinase domain and a sterile-α-motif (SAM) domain are present in the intracellular region on the C-terminal side thereof.

As a ligand of EPHA2, Ephrin-Al to A5 of GPI-anchored protein have been known (Non Patent Literature 2). As a result of activation of the tyrosine kinase domain of EPHA2 caused by ligand binding, tyrosine residues existing in the intracellular region of EPHA2 are auto-phosphorylated, and intracellular signal transduction is thereby induced. In addition, it has been reported that, after EPHA2 has bound to a ligand, it is incorporated into cells according to endocytosis, and is finally decomposed in the proteasome (Non Patent Literature 3).

It has been reported that EPHA2 is clinically expressed at a high level in many cancer species, and in particular, in breast cancer, esophageal cancer, prostate cancer, gastric cancer, non-small-cell lung cancer, colon cancer, and glioblastoma multiforme (Non Patent Literatures 4, 5, 6, 7, 8, 9, 10, and 11). Moreover, in esophageal cancer, a significant correlation has been observed between the expression of EPHA2, and regional lymph node metastasis, the number of lymph node metastasis and a low degree of differentiation of cancer, and it has been reported that the survival rate of EPHA2-positive patients is lower than that of EPHA2-negative patients (Non Patent Literature 5). It has been reported that, in non-small-cell lung cancer, the expression level of EPHA2 is higher in patients having a disease-free survival period of less than 5 years than in patients having a disease-free survival period of longer than 5 years, that the expression level of EPHA2 is higher in relapsed patients than in non-relapsed patients, and that the expression level of EPHA2 is higher in patients with brain metastasis than in non-relapsed patients or patients in whom metastasis is limited to the other lung (Non Patent Literature 9). It has been reported that, even in colon cancer, the expression level of EPHA2 is significantly correlated with liver metastasis, lymphatic invasion, and clinical stage (Non Patent Literature 10).

Also, it has been reported that non-cancer cells acquire cancer traits, such as anchorage independent proliferative ability, ability to form a tubular form on the extracellular matrix, and *in vivo* tumor proliferative ability, by introducing an EPHA2 gene into the cells (Non Patent Literature 4), and that the invasiveness of cancer cells into the extracellular matrix is accelerated (Non Patent Literatures 12 and 13). It has also reported that, to the contrary, if the expression of EPHA2 is suppressed by siRNA, the invasiveness of cancer cells, anchorage independent proliferation thereof, and *in vivo* tumor proliferation are suppressed (Non Patent Literatures 13 and 14), and that the invasiveness of cancer cells, anchorage independent proliferation thereof, and tubular form-forming ability thereof are suppressed by activating EPHA2 using a fusion protein of Ephrin-Al serving as a ligand and the Fc region of human IgG, and thus inducing the decomposition of EPHA2 according to endocytosis (Non Patent Literatures 4, 11, and 12).

On the other hand, it has been reported that EPHA2 is expressed not only in cancer cells, but also in tumor or in blood vessels existing around the tumor (Non Patent Literature 15). It has been reported that, in the case of mice, EPHA2 signals are associated with angiogenesis induced by Ephrin-Al, and that EPHA2, which is particularly expressed in vascular endothelial cells, is necessary for the lumen formation of the vascular endothelial cells or the survival thereof (Non Patent Literature 16). It has been further reported that a fusion protein of the extracellular region of EPHA2 and the Fc region of human IgG suppresses angiogenesis *in vivo* and exhibits antitumor effects (Non Patent Literature 17).

Recently, it has been reported that membrane type-1 matrix metalloprotease (MT1-MMP) cleaves EPHA2 at the extracellular region, and as a result, an EPAH2 fragment remaining in the membrane promotes carcinogenic signaling (Non Patent Literatures 18 and 19).

Furthermore, it has been reported that the serum concentration of soluble EPHA2 is higher in patients with lung cancer or prostate cancer than in healthy donors (Non Patent Literatures 20 and 21).

As described above, it has been suggested that EPHA2 is likely to be an excellent therapeutic target in cancer, and as a matter of fact, several clinical tests of drugs that target EPHA2 have actually been carried out (Patent Literatures 1 to 3).

From these things, provision of a method capable of detecting the expression of EPHA2 is useful for the examination or diagnosis of diseases associated with EPHA2, such as cancer, and the expression of EPHA2. Immunoassays are methods for measuring EPHA2 contained in a sample derived from a subject. Among them, an ELISA (Enzyme-Linked Immuno Sorbent Assay) method has been commonly used as a simple and prompt measurement method. To date, a method of measuring EPHA2 contained in a test sample using an anti-EPHA2 antibody (ELISA method) has been reported (Patent Literatures 4 and 5 and Non Patent Literatures 20 to 27), but a method of using an anti-EPHA2 monoclonal antibody alone has not yet been established.

### Citation List

### Patent Literature

Patent Literature 1: International Publication No. WO2003/094859
Patent Literature 2: International Publication No. WO2006/084226
Patent Literature 3: International Publication No. WO2009/028639
Patent Literature 4: International Publication No. WO2009/085805
Patent Literature 5: International Publication No. WO2014/126160

### Non Patent Literature

Non Patent Literature 1: Molecular and Cellular Biology, 1990, Vol. 10, pp. 6316-6324
Non Patent Literature 2: Annual Review of Neuroscience, 1998, Vol. 21, pp. 309-345
Non Patent Literature 3: Molecular Cancer Research, 2002, Vol. 1, pp. 79-87
Non Patent Literature 4: Cancer Research, 2001, Vol. 61, pp. 2301-2306
Non Patent Literature 5: International Journal of Cancer, 2003, Vol. 103, pp. 657-663
Non Patent Literature 6: The Prostate, 1999, Vol. 41, pp. 275-280
Non Patent Literature 7: American Journal of Pathology, 2003, Vol. 163, pp. 2271-2276
Non Patent Literature 8: Cancer Science, 2005, Vol. 96, pp. 42-47
Non Patent Literature 9: Clinical Cancer Research, 2003, Vol. 9, pp. 613-618
Non Patent Literature 10: Oncology Reports, 2004, Vol. 11, pp. 605-611
Non Patent Literature 11: Molecular Cancer Research, 2005, Vol. 3, pp. 541-551
Non Patent Literature 12: Biochemical and Biophysical Research Communications, 2004, Vol. 320, pp. 1096-1102
Non Patent Literature 13: Oncogene, 2004, Vol. 23, pp. 1448-1456
Non Patent Literature 14: Cancer Research, 2002, Vol. 62, pp. 2840-2847
Non Patent Literature 15: Oncogene, 2000, Vol. 19, pp. 6043-6052
Non Patent Literature 16: Journal of Cell Science, 2004, Vol. 117, pp. 2037-2049
Non Patent Literature 17: Oncogene, 2002, Vol. 21, pp. 7011-7026
Non Patent Literature 18: The Journal of Cell Biology, 2013, Vol. 201, pp. 467-484
Non Patent Literature 19: Cancer Research, 2015, Vol. 75, pp. 3327-3339
Non Patent Literature 20: Proceedings: AACR 106th Annual Meeting 2015; April 18-22, 2015; Philadelphia, PA Cancer Research 2015; 75 (15 Suppl): Abstract nr 546.
Non Patent Literature 21: The 74th Annual Meeting of the Japanese Cancer Association, P15-8 P-3321
Non Patent Literature 22: RayBiotech, Human EphA2 ELISA (ELH-EPHA2-1), <URL:http://www.raybiotech.com/epha2-elisa.html>
Non Patent Literature 23: CUSABIO, Human Ephrin type-A receptor 2(EPHA2) ELISA kit (CSB-EL007722HU), <http://www.cusabio.com/ELISA-Kit/Human-Ephrin-type-A-receptor-2EPHA2-ELISA-kit-76651.html>
Non Patent Literature 24: LSBio, Human EPHA2 / EPH Receptor A2 ELISA Kit (Sandwich ELISA) (LS-F23977), <https://www.lsbio.com/elisakits/human-epha2-eph-receptor-a2-elisa-kit-sandwich-elisa-ls-f23977/23977?trid=119>
Non Patent Literature 25: Abnova, EPHA2 (Human) Matched Antibody Pair (H00001969-AP21), <http://www.abnova.com/products/products_detail.asp?catalog_id=H00001969-AP21>
Non Patent Literature 26: Abnova, EPHA2 (Human) Matched Antibody Pair (H00001969-AP11), <http://www.abnova.com/products/products_detail.asp?catalog_id=H00001969-AP11>
Non Patent Literature 27: Abnova, EPHA2 (Human) Matched Antibody Pair (H00001969-AP51), <http://www.abnova.com/products/products_detail.asp?catalog_id=H00001969-AP51>

### Summary of Invention

### Technical Problem

It is one object of the present invention to provide an antibody specific to EPHA2.

It is another object of the present invention to provide a composition for immunological diagnosis, comprising an anti-EPHA2 antibody, and anti-EPHA2 antibodies used therefor or a combination thereof.

Moreover, it is a further object of the present invention to provide a method for detecting EPHA2, using anti-EPHA2 antibodies or a combination thereof, and a kit for detection or diagnosis, comprising the antibodies or a combination thereof.

### Solution to Problem

For the purpose of simply measuring EPHA2 with high sensitivity and utilizing such a simple measurement for to improve the accuracy of cancer diagnosis, the present inventors have produced a large number of anti-EPHA2 monoclonal antibodies for the measurement of EPHA2 according to a sandwich immunoassay, have then selected monoclonal antibodies particularly suitable for the sandwich immunoassay, have then determined the amino acid sequence and the nucleotide sequence of a heavy chain variable region and a light chain variable region thereof, and have further analyzed six CDR sequences.

The present inventors have confirmed that, by using two types of antibodies developed by the present inventors, EPHA2 in a biological sample can be measured with high sensitivity and detection of cancer can also be achieved with high sensitivity and high specificity, thereby completing the present invention.

Specifically, the present invention is as follows.
[1] A monoclonal antibody specifically binding to EPHA2, comprising:
   (a) a heavy chain CDRH1 consisting of the amino acid sequence as set forth in SEQ ID NO: 29,
   (b) a heavy chain CDRH2 consisting of the amino acid sequence as set forth in SEQ ID NO: 30,
   (c) a heavy chain CDRH3 consisting of the amino acid sequence as set forth in SEQ ID NO: 31,
   (d) a light chain CDRL1 consisting of the amino acid sequence as set forth in SEQ ID NO: 32,
   (e) a light chain CDRL2 consisting of the amino acid sequence as set forth in SEQ ID NO: 33, and
   (f) a light chain CDRL3 consisting of the amino acid sequence as set forth in SEQ ID NO: 34, or
      a functional fragment of the monoclonal antibody specifically binding to EPHA2.
[2] A monoclonal antibody specifically binding to EPHA2, comprising a heavy chain variable region consisting of the amino acid sequence as set forth in SEQ ID NO: 20 and a light chain variable region consisting of the amino acid sequence as set forth in SEQ ID NO: 22, or a functional fragment of the monoclonal antibody specifically binding to EPHA2.
[3] A monoclonal antibody specifically binding to EPHA2, comprising: a heavy chain variable region consisting of an amino acid sequence having a sequence identity of 95% or more to the amino acid sequence as set forth in SEQ ID NO: 20, and having binding activity to human EPHA2; and a light chain variable region consisting of an amino acid sequence having a sequence identity of 95% or more to the amino acid sequence as set forth in SEQ ID NO: 22, and having binding activity to human EPHA2, or a functional fragment of the monoclonal antibody specifically binding to EPHA2.
[4] A monoclonal antibody specifically binding to EPHA2, comprising:
   (a) a heavy chain CDRH1 consisting of the amino acid sequence as set forth in SEQ ID NO: 23,
   (b) a heavy chain CDRH2 consisting of the amino acid sequence as set forth in SEQ ID NO: 24,
   (c) a heavy chain CDRH3 consisting of the amino acid sequence as set forth in SEQ ID NO: 25,
   (d) a light chain CDRL1 consisting of the amino acid sequence as set forth in SEQ ID NO: 26,
   (e) a light chain CDRL2 consisting of the amino acid sequence as set forth in SEQ ID NO: 27, and
   (f) a light chain CDRL3 consisting of the amino acid sequence as set forth in SEQ ID NO: 28, or
      a functional fragment of the monoclonal antibody specifically binding to EPHA2.
[5] A monoclonal antibody specifically binding to EPHA2, comprising a heavy chain variable region consisting of the amino acid sequence as set forth in SEQ ID NO: 15 and a light chain variable region consisting of the amino acid sequence as set forth in SEQ ID NO: 18, or a functional fragment of the monoclonal antibody specifically binding to EPHA2.
[6] A monoclonal antibody specifically binding to EPHA2, comprising: a heavy chain variable region consisting of an amino acid sequence having a sequence identity of 95% or more to the amino acid sequence as set forth in SEQ ID NO: 15, and having binding activity to human EPHA2; and a light chain variable region consisting of an amino acid sequence having a sequence identity of 95% or more to the amino acid sequence as set forth in SEQ ID NO: 18, and having binding activity to human EPHA2, or a functional fragment of the monoclonal antibody specifically binding to EPHA2.
[7] A combination of two types of monoclonal antibodies; namely, the monoclonal antibody specifically binding to EPHA2 according to any one of the above [1] to [3], or a functional fragment of the monoclonal antibody specifically binding to EPHA2, and the monoclonal antibody specifically binding to EPHA2, or a functional fragment of the monoclonal antibody specifically binding to EPHA2 according to any one of the above [4] to [6].
[8] The combination according to the above [7], which is used in a method for detecting or measuring EPHA2 in a biological sample.
[9] A method for specifically detecting EPHA2 contained in a biological sample according to an immunoassay, which uses the monoclonal antibody specifically binding to EPHA2, or a functional fragment of the monoclonal antibody specifically binding to EPHA2 according to any one of the above [1] to [6].
[10] A method for specifically detecting EPHA2 contained in a biological sample according to a sandwich immunoassay, which uses two types of monoclonal antibodies; namely, the monoclonal antibody specifically binding to EPHA2, or a functional fragment of the monoclonal antibody specifically binding to EPHA2 according to any one of the above [1] to [3], and the monoclonal antibody specifically binding to EPHA2, or a functional fragment of the monoclonal antibody specifically binding to EPHA2. according to any one of the above [4] to [6]
[11] The method according to the above [10], wherein the sandwich immunoassay is ELISA.
[12] A method for detecting cancer, which comprises: measuring the concentration of EPHA2 in the blood of a subject according to an immunoassay, using the monoclonal antibody specifically binding to EPHA2, or a functional fragment of the monoclonal antibody specifically binding to EPHA2 according to any one of the above [1] to [6]; and evaluating that the subject suffers from cancer, when the concentration of EPHA2 in the blood of the subject is higher than the concentration of EPHA2 in the blood of a healthy donor.
[13] A method for detecting cancer, which comprises: measuring the concentration of EPHA2 in the blood of a subject according to a sandwich immunoassay, using two types of monoclonal antibodies, namely, the monoclonal antibody specifically binding to EPHA2, or a functional fragment of the monoclonal antibody specifically binding to EPHA2 according to any one of the above [1] to [3], and the monoclonal antibody specifically binding to EPHA2, or a functional fragment of the monoclonal antibody specifically binding to EPHA2 according to any one of the above [4] to [6]; and evaluating that the subject suffers from cancer, when the concentration of EPHA2 in the blood of the subject is higher than the concentration of EPHA2 in the blood of a healthy donor.
[14] The method according to the above [13], wherein the sandwich immunoassay is ELISA.
[15] The method according to any one of the above [12] to [14], wherein the cancer is selected from the group consisting of gastric cancer, ovarian cancer, breast cancer, esophageal cancer, prostate cancer, non-small-cell lung cancer, colon cancer, pancreatic cancer, liver cancer, bladder cancer, cervical cancer, uterine cancer, kidney cancer, brain tumor, malignant melanoma, and head and neck cancer.
[16] A kit for measuring EPHA2, comprising two types of monoclonal antibodies or functional fragments thereof; namely, the monoclonal antibody specifically binding to EPHA2, or a functional fragment of the monoclonal antibody specifically binding to EPHA2 according to any one of the above [1] to [3], and the monoclonal antibody specifically binding to EPHA2, or a functional fragment of the monoclonal antibody specifically binding to EPHA2 according to any one of the above [4] to [6].
[17] A kit for detecting cancer, comprising two types of monoclonal antibodies or functional fragments thereof; namely, the monoclonal antibody specifically binding to EPHA2, or a functional fragment of the monoclonal antibody specifically binding to EPHA2 according to any one of the above [1] to [3], and the monoclonal antibody specifically binding to EPHA2, or a functional fragment of the monoclonal antibody specifically binding to EPHA2 according to any one of the above [4] to [6].
[18] The kit according to the above [17], wherein the cancer is selected from the group consisting of gastric cancer, ovarian cancer, breast cancer, esophageal cancer, prostate cancer, non-small-cell lung cancer, colon cancer, pancreatic cancer, liver cancer, bladder cancer, cervical cancer, uterine cancer, kidney cancer, brain tumor, malignant melanoma, and head and neck cancer.

The present description includes part or all of the contents as disclosed in Japanese Patent Application No. 2017-009349, which is a priority document of the present application.

### Advantageous Effects of Invention

Among a large number of anti-EPHA2 monoclonal antibodies produced by the present inventors, when the antibody 57-1 and the antibody 230-1 are used in combination as a solid-phase antibody and a detection antibody, EPHA2 in a biological sample can be detected with higher sensitivity than in the case of using other monoclonal antibodies. Moreover, when EPHA2 is measured and cancer is detected according to a sandwich immunoassay using the above-described two types of monoclonal antibodies in combination, the cancer can be detected with high sensitivity and high specificity.

Furthermore, EPHA2 in a biological sample can be detected with higher sensitivity according to an immunoassay using the antibody 57-1 or the antibody 230-1, than in the case of using other monoclonal antibodies. Further, when EPHA2 is measured and cancer is detected according to an immunoassay using the above-described monoclonal antibodies, the cancer can be detected with high sensitivity and high specificity.

### Brief Description of Drawings

[Figure 1] Figure 1 is a view showing the reactivity of the 13 types of anti-EPHA2 monoclonal antibodies (SHM41, 57-1, 80-21, 83-21, 113-1, 119-1, 131-1, 141-21, 148-1, 151-4, 230-1, 240-4, and 245-21) according to Example 2)-1, to EPHA2.
[Figure 2] Figure 2 is a view showing reactivity in the case of using the anti-EPHA2 monoclonal antibody 230-1 as a biotin-labeled antibody according to Example 2)-2, and using 11 types of anti-EPHA2 monoclonal antibodies 57-1, 80-21, 83-21, 113-1, 119-1, 131-1, 141-21, 148-1, 151-4, 240-4, and 245-21 as capture antibodies.
[Figure 3] Figure 3 is a view showing reactivity in the case of using SHM41, 57-1 or 230-1 as a capture antibody according to Example 2)-3, and using biotin-labeled 57-1 or 230-1 as a detection antibody.
[Figure 4] Figure 4 is a view showing the reactivity of the ELISA system according to Example 2)-4 (the capture antibody 230-1, the detection antibody biotin-labeled 57-1, the capture antibody 57-1, and the detection antibody biotin-labeled 230-1) to EPHA2.
[Figure 5] Figure 5 is a view showing the calibration curve of the ELISA system according to Example 2)-5 (the capture antibody 230-1 and the detection antibody biotin-labeled 57-1).
[Figure 6] Figure 6 is a view showing the EPHA2 specificity of the ELISA system according to Example 2)-6 (the capture antibody 230-1 and the detection antibody biotin-labeled 57-1).
[Figure 7-1] Figure 7-1 is a view showing the measurement of the serum concentration of EPHA2 of healthy donors, gastric cancer patients, and ovarian cancer patients according to the ELISA system (the capture antibody 230-1 and the detection antibody biotin-labeled 57-1).
[Figure 7-2] Figure 7-2 is a view showing the measurement of the serum concentration of EPHA2 of healthy donors and non-small-cell lung cancer patients according to the ELISA system (the capture antibody 230-1 and the detection antibody biotin-labeled 57-1).
[Figure 8] Figure 8 is a view showing the nucleotide sequence (SEQ ID NO: 14) of cDNA encoding the heavy chain variable region of the mouse anti-EPHA2 monoclonal antibody 57-1.
[Figure 9] Figure 9 is a view showing the amino acid sequence (SEQ ID NO: 15) of the heavy chain variable region of the mouse anti-EPHA2 monoclonal antibody 57-1.
[Figure 10] Figure 10 is a view showing the nucleotide sequence (SEQ ID NO: 17) of cDNA encoding the light chain variable region of the mouse anti-EPHA2 monoclonal antibody 57-1.
[Figure 11] Figure 11 is a view showing the amino acid sequence (SEQ ID NO: 18) of the light chain variable region of the mouse anti-EPHA2 monoclonal antibody 57-1.
[Figure 12] Figure 12 is a view showing the nucleotide sequence (SEQ ID NO: 19) of cDNA encoding the heavy chain variable region of the mouse anti-EPHA2 monoclonal antibody 230-1.
[Figure 13] Figure 13 is a view showing the amino acid sequence (SEQ ID NO: 20) of the heavy chain variable region of the mouse anti-EPHA2 monoclonal antibody 230-1.
[Figure 14] Figure 14 is a view showing the nucleotide sequence (SEQ ID NO: 21) of cDNA encoding the light chain variable region of the mouse anti-EPHA2 monoclonal antibody 230-1.
[Figure 15] Figure 15 is a view showing the amino acid sequence (SEQ ID NO: 22) of the light chain variable region of the mouse anti-EPHA2 monoclonal antibody 230-1.
[Figure 16] Figure 16 is a view showing the amino acid sequences (SEQ ID NOS: 23 to 28) of the CDRs of the mouse anti-EPHA2 monoclonal antibody 57-1.
[Figure 17] Figure 17 is a view showing the amino acid sequences (SEQ ID NOS: 29 to 34) of the CDRs of the mouse anti-EPHA2 monoclonal antibody 230-1.

### Description of Embodiments

Hereinafter, the present invention will be described in detail.

The present invention relates to a method for measuring EPHA2 in the biological sample of a subject according to an immunoassay of using a specific monoclonal antibody specifically binding to EPHA2, and a reagent or a kit for use in the measurement.

In addition, the present invention relates to a method for measuring EPHA2 in the biological sample of a subject according to a sandwich immunoassay of using two types of specific monoclonal antibodies specifically binding to EPHA2, and a reagent or a kit for use in the measurement.

Moreover, the present invention relates to anti-EPHA2 monoclonal antibodies suitable for the measurement of EPHA2 according to a sandwich immunoassay, and a combination of two types of anti-EPHA2 monoclonal antibodies.

Furthermore, the present invention relates to: a method comprising measuring EPHA2 in the biological sample of a subject (preferably, in blood) according to an immunoassay of using a specific anti-EPHA2 monoclonal antibody, and then detecting whether or not the subject suffers from cancer, using the concentration of EPHA2 in the biological sample (preferably, in the blood) as an indicator; and a reagent or a kit for detecting whether or not the subject suffers from cancer, using the concentration of EPHA2 in the biological sample (preferably, in the blood) as an indicator. The method of the present invention is also a method for obtaining auxiliary data used when EPHA2 in the biological sample of a subject (preferably, in blood) is measured according to an immunoassay of using a specific anti-EPHA2 monoclonal antibody, and then, whether or not the subject suffers from cancer is determined by using the concentration of EPHA2 in the biological sample (preferably, in the blood) as an indicator.

Further, the present invention relates to a method comprising measuring EPHA2 in the biological sample of a subject (preferably, in blood) according to a sandwich immunoassay of two types of specific anti-EPHA2 monoclonal antibodies, and then detecting whether or not the subject suffers from cancer, using the concentration of EPHA2 in the biological sample (preferably, in the blood) as an indicator; and a reagent or a kit for detecting whether or not the subject suffers from cancer, using the concentration of EPHA2 in the biological sample (preferably, in the blood). The method of the present invention is also a method for obtaining auxiliary data used when EPHA2 in the biological sample of a subject (preferably, in blood) is measured according to a sandwich immunoassay of using two types of specific anti-EPHA2 monoclonal antibodies, and then, whether or not the subject suffers from cancer is determined by using the concentration of EPHA2 in the biological sample (preferably, in the blood) as an indicator.

As a biological sample, blood (whole blood, serum, or plasma), lymph, urine, a tissue homogenate, a cell culture supernatant, or a cell lysate is used. Preferably, blood may be used, and more preferably, serum may be used.

The monoclonal antibody used in the present invention can be obtained by immunizing a mammal such as a horse, a monkey, a dog, a pig, a bovine, a goat, sheep, a rabbit, a rat, a Guinea pig, a hamster or a mouse with an immunogen that is EPHA2 or a fragment thereof, then fusing splenic cells or the like with myelomas to produce hybridomas, and then obtaining an antibody as a result of generation and secretion from the hybridomas. Hybridomas can be produced by a known method.

EPHA2 used as an immunogen can be chemically synthesized based on sequence information, or can also be obtained as a recombinant protein according to a known method, based on the information of the DNA sequence encoding the protein.

The antibody can be screened according to any given method. The antibody may be preferably screened according to Cell-ELISA using animal cells that are transfected with DNA encoding EPHA2. The nucleotide sequence of EPHA2 is as set forth in SEQ ID NO: 5, and the amino acid sequence of EPHA2 is as set forth in SEQ ID NO: 6. The specific binding ability of the selected antibody to EPHA2 can be confirmed by a flow cytometric analysis, etc.

The monoclonal antibody used in the method of the present invention is either one or both of the antibody 230-1 and the antibody 57-1. The antibody 230-1 is a monoclonal antibody having the highest reactivity with EPHA2 among the produced 13 types of monoclonal antibodies. On the other hand, the antibody 57-1 is most suitable for being combined with the antibody 230-1 for use in the measurement of EPHA2 according to a sandwich immunoassay.

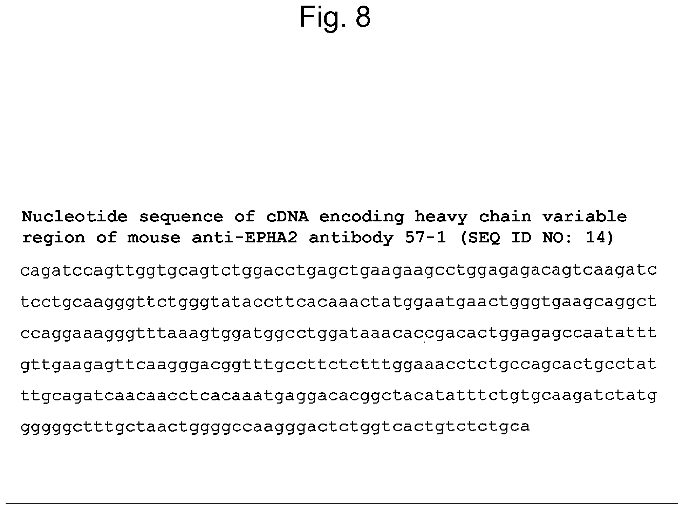

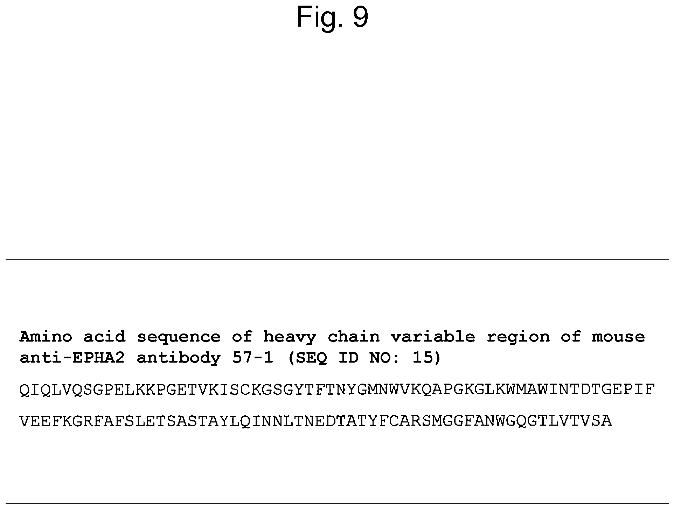

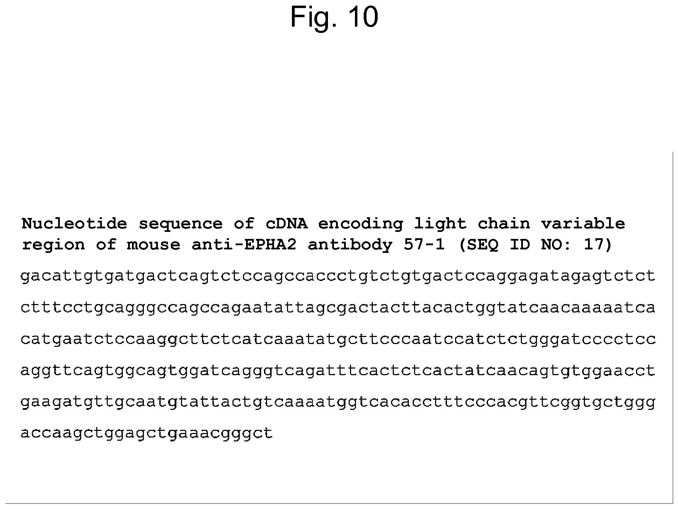

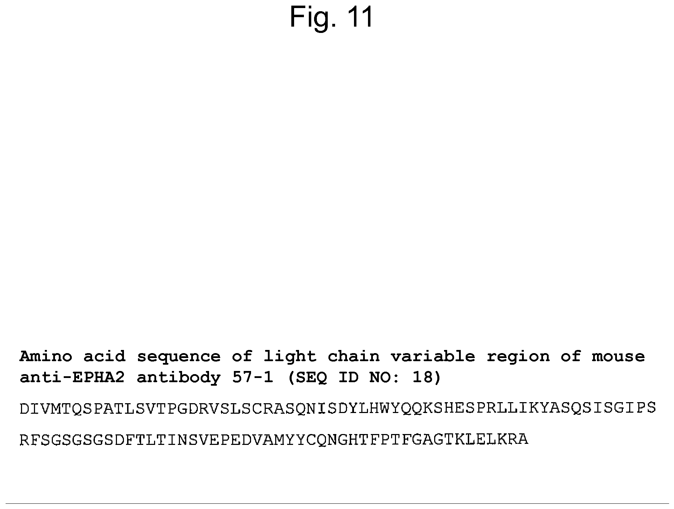

The cDNA sequence encoding the heavy chain variable region of the antibody 57-1 is as set forth in SEQ ID NO: 14 (Figure 8), and the amino acid sequence of the heavy chain variable region of the antibody 57-1 is as set forth in SEQ ID NO: 15 (Figure 9). Moreover, the cDNA sequence encoding the light chain variable region of the antibody 57-1 is as set forth in SEQ ID NO: 17 (Figure 10), and the amino acid sequence of the light chain variable region of the antibody 57-1 is as set forth in SEQ ID NO: 18 (Figure 11).

Specifically, the anti-EPHA2 antibody of the present invention is an anti-human EPHA2 monoclonal antibody binding to human EPHA2, which comprises a heavy chain variable region consisting of the amino acid sequence as set forth in SEQ ID NO: 15 and a light chain variable region consisting of the amino acid sequence as set forth in SEQ ID NO: 18.

In addition, DNA, which consists of a nucleotide sequence having a sequence identity of at least 85% or more, preferably 90% or more, more preferably 95% or more, particularly preferably 97% or more, 98% or more, or 99% or more, to the nucleotide sequence as set forth in the above-described SEQ ID NO: 14 or 17, in which the sequence identity is calculated using BLAST (Basic Local Alignment Search Tool at the National Center for Biological Information), etc. (for example, default, namely, initial setting parameters), and which encodes a protein having the activity of the heavy chain variable region or light chain variable region of an antibody, namely, binding activity to human EPHA2, is also included in the DNA encoding the heavy chain variable region or light chain variable region of the antibody of the present invention.

Moreover, DNA, which can hybridize under stringent conditions with DNA consisting of a sequence complementary to the DNA consisting of the sequence as set forth in the above-described SEQ ID NO: 14 or 17, and which encodes a protein having the activity of the heavy chain variable region or light chain variable region of an antibody, namely, binding activity to human EPHA2, is also included in the DNA encoding the heavy chain variable region or light chain variable region of the present invention.

Furthermore, the above-described heavy chain variable region or light chain variable region does not only include the heavy chain variable region or light chain variable region consisting of the amino acid sequence as set forth in SEQ ID NO: 15 or 18, but also includes a heavy chain variable region or a light chain variable region, which consists of an amino acid sequence comprising a deletion, substitution or addition of one or several, for example, 1 to 10, preferably 1 to 5, more preferably 1 or 2, and further preferably 1 amino acid, with respect to the amino acid sequence as set forth in SEQ ID NO: 15 or 18, and which has the activity of the heavy chain variable region or light chain variable region of an antibody, namely, binding activity to human EPHA2.

An example of the amino acid sequence comprising a deletion, substitution or addition of one or several amino acids, with respect to the amino acid sequence as set forth in SEQ ID NO: 15 or 18, may be an amino acid sequence having a sequence identity of at least 85% or more, preferably 90% or more, more preferably 95% or more, particularly preferably 97% or more, 98% or more, or 99% or more, to the amino acid sequence as set forth in SEQ ID NO: 15 or 18, when the sequence identity is calculated using BLAST (Basic Local Alignment Search Tool at the National Center for Biological Information), etc. (for example, default, namely, initial setting parameters).

Such a protein having an amino acid sequence comprising a deletion, substitution or addition of one or several amino acids, with respect to the amino acid sequence as set forth in SEQ ID NO: 15 or 18, is substantially identical to the protein having the amino acid sequence as set forth in SEQ ID NO: 15 or 18.

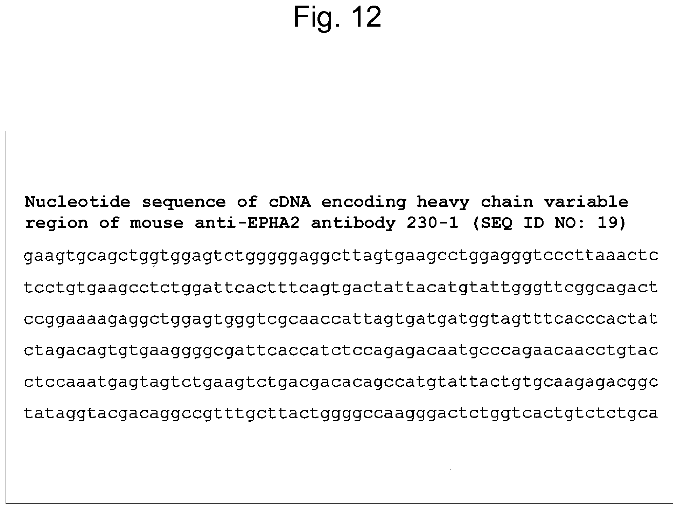

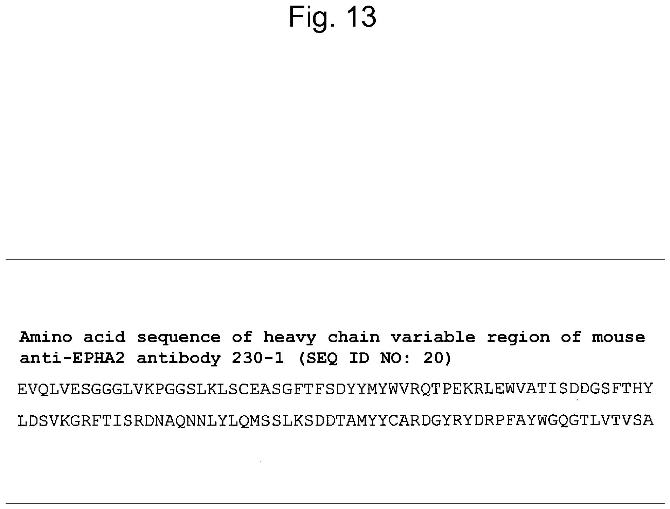

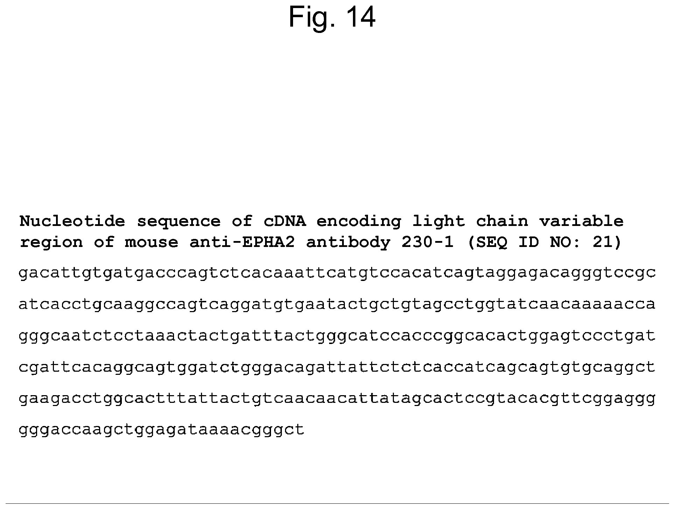

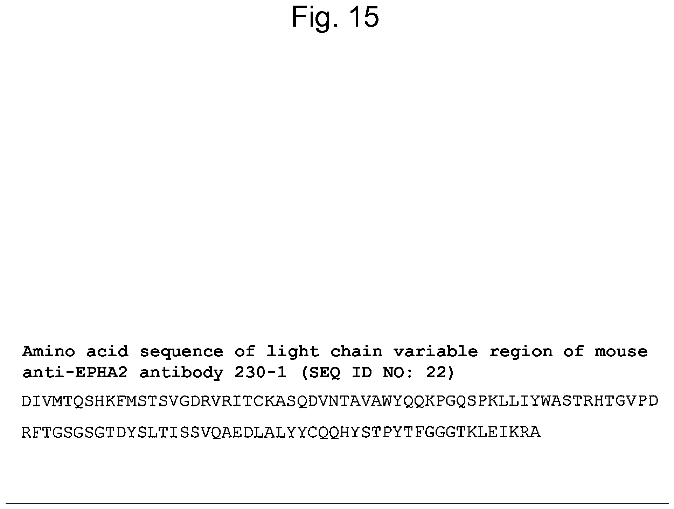

The cDNA sequence encoding the heavy chain variable region of the antibody 230-1 is as set forth in SEQ ID NO: 19 (Figure 12), and the amino acid sequence of the heavy chain variable region of the antibody 230-1 is as set forth in SEQ ID NO: 20 (Figure 13). Moreover, the cDNA sequence encoding the light chain variable region of the antibody 230-1 is as set forth in SEQ ID NO: 21 (Figure 14), and the amino acid sequence of the light chain variable region of the antibody 230-1 is as set forth in SEQ ID NO: 22 (Figure 15).

Specifically, the anti-EPHA2 antibody of the present invention is an anti-human EPHA2 monoclonal antibody binding to human EPHA2, which comprises a heavy chain variable region consisting of the amino acid sequence as set forth in SEQ ID NO: 20 and a light chain variable region consisting of the amino acid sequence as set forth in SEQ ID NO: 22.

In addition, DNA, which consists of a nucleotide sequence having a sequence identity of at least 85% or more, preferably 90% or more, more preferably 95% or more, particularly preferably 97% or more, 98% or more, or 99% or more, to the nucleotide sequence as set forth in the above-described SEQ ID NO: 19 or 21, in which the sequence identity is calculated using BLAST (Basic Local Alignment Search Tool at the National Center for Biological Information), etc. (for example, default, namely, initial setting parameters), and which encodes a protein having the activity of the heavy chain variable region or light chain variable region of an antibody, namely, binding activity to human EPHA2, is also included in the DNA encoding the heavy chain variable region or light chain variable region of the antibody of the present invention.

Moreover, DNA, which can hybridize under stringent conditions with DNA consisting of a sequence complementary to the DNA consisting of the sequence as set forth in the above-described SEQ ID NO: 19 or 21, and which encodes a protein having the activity of the heavy chain variable region or light chain variable region of an antibody, namely, binding activity to human EPHA2, is also included in the DNA encoding the heavy chain variable region or light chain variable region of the present invention.

Furthermore, the above-described heavy chain variable region or light chain variable region does not only include the heavy chain variable region or light chain variable region consisting of the amino acid sequence as set forth in SEQ ID NO: 20 or 22, but also includes a heavy chain variable region or a light chain variable region, which consists of an amino acid sequence comprising a deletion, substitution or addition of one or several, for example, 1 to 10, preferably 1 to 5, more preferably 1 or 2, and further preferably 1 amino acid, with respect to the amino acid sequence as set forth in SEQ ID NO: 20 or 22, and which has the activity of the heavy chain variable region or light chain variable region of an antibody, namely, binding activity to human EPHA2.

An example of the amino acid sequence comprising a deletion, substitution or addition of one or several amino acids, with respect to the amino acid sequence as set forth in SEQ ID NO: 20 or 22, may be an amino acid sequence having a sequence identity of at least 85% or more, preferably 90% or more, more preferably 95% or more, particularly preferably 97% or more, 98% or more, or 99% or more, to the amino acid sequence as set forth in SEQ ID NO: 20 or 22, when the sequence identity is calculated using BLAST (Basic Local Alignment Search Tool at the National Center for Biological Information), etc. (for example, default, namely, initial setting parameters).

Such a protein having an amino acid sequence comprising a deletion, substitution or addition of one or several amino acids, with respect to the amino acid sequence as set forth in SEQ ID NO: 20 or 22, is substantially identical to the protein having the amino acid sequence as set forth in SEQ ID NO: 20 or 22.

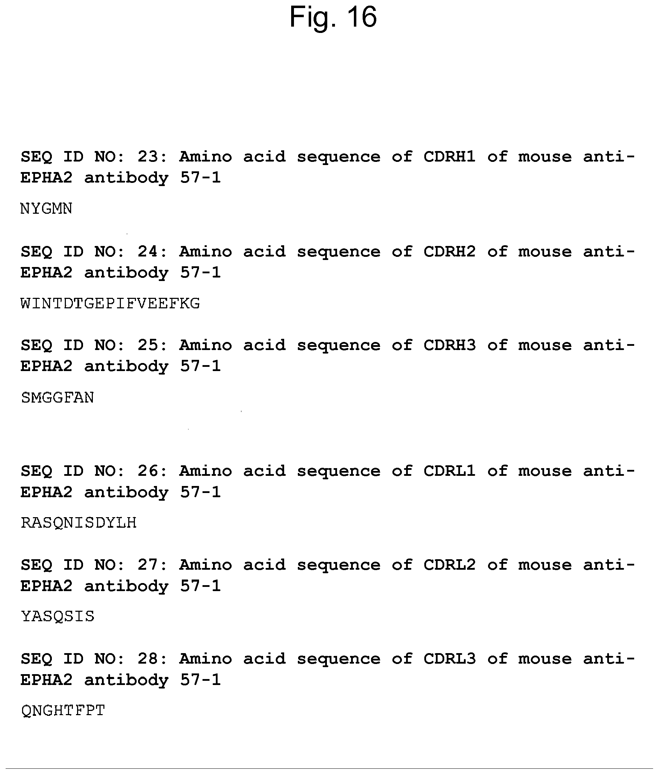

Further, the antibody 57-1 comprises, as CDRs (complementarity determining regions) of the heavy chain variable region, CDRH1 consisting of the amino acid sequence as set forth in SEQ ID NO: 23 (NYGMN), CDRH2 consisting of the amino acid sequence as set forth in SEQ ID NO: 24 (WINTDTGEPIFVEEFKG), and CDRH3 consisting of the amino acid sequence as set forth in SEQ ID NO: 25 (SMGGFAN), and further comprises, as CDRs of the light chain variable region, CDRL1 consisting of the amino acid sequence as set forth in SEQ ID NO: 26 (RASQNISDYLH), CDRL2 consisting of the amino acid sequence as set forth in SEQ ID NO: 27 (YASQSIS), and CDRL3 consisting of the amino acid sequence as set forth in SEQ ID NO: 28 (QNGHTFPT) (Figure 16).

Specifically, the anti-EPHA2 antibody of the present invention is an antibody comprising the heavy chain variable region CDRH1 consisting of the amino acid sequence as set forth in SEQ ID NO: 23, the heavy chain variable region CDRH2 consisting of the amino acid sequence as set forth in SEQ ID NO: 24, the heavy chain variable region CDRH3 consisting of the amino acid sequence as set forth in SEQ ID NO: 25, the light chain variable region CDRL1 consisting of the amino acid sequence as set forth in SEQ ID NO: 26, the light chain variable region CDRL2 consisting of the amino acid sequence as set forth in SEQ ID NO: 27, and the light chain variable region CDRL3 consisting of the amino acid sequence as set forth in SEQ ID NO: 28.

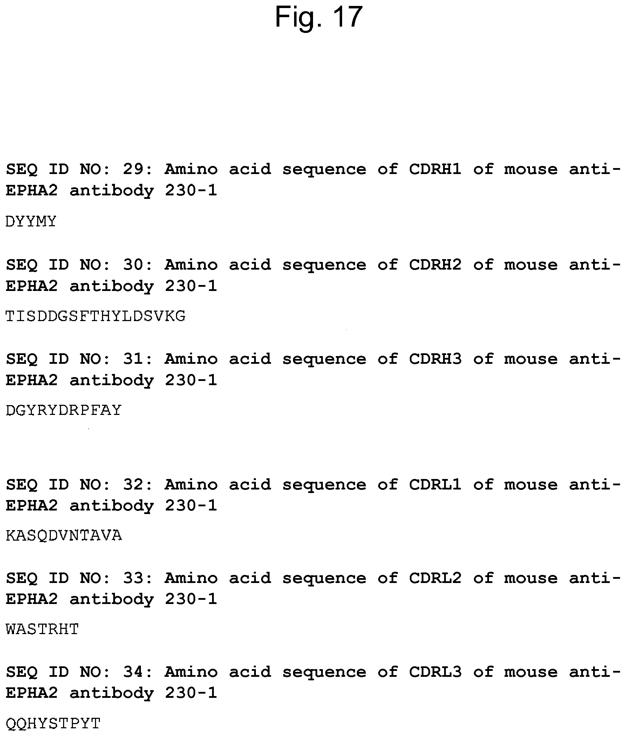

Still further, the antibody 230-1 comprises, as CDRs (complementarity determining regions) of the heavy chain variable region, CDRH1 consisting of the amino acid sequence as set forth in SEQ ID NO: 29 (DYYMY), CDRH2 consisting of the amino acid sequence as set forth in SEQ ID NO: 30 (TISDDGSFTHYLDSVKG), and CDRH3 consisting of the amino acid sequence as set forth in SEQ ID NO: 31 (DGYRYDRPFAY), and further comprises, as CDRs of the light chain variable region, CDRL1 consisting of the amino acid sequence as set forth in SEQ ID NO: 32 (KASQDVNTAVA), CDRL2 consisting of the amino acid sequence as set forth in SEQ ID NO: 33 (WASTRHT), and CDRL3 consisting of the amino acid sequence as set forth in SEQ ID NO: 34 (QQHYSTPYT) (Figure 17).

Specifically, the anti-EPHA2 antibody of the present invention is an antibody comprising the heavy chain variable region CDRH1 consisting of the amino acid sequence as set forth in SEQ ID NO: 29, the heavy chain variable region CDRH2 consisting of the amino acid sequence as set forth in SEQ ID NO: 30, the heavy chain variable region CDRH3 consisting of the amino acid sequence as set forth in SEQ ID NO: 31, the light chain variable region CDRL1 consisting of the amino acid sequence as set forth in SEQ ID NO: 32, the light chain variable region CDRL2 consisting of the amino acid sequence as set forth in SEQ ID NO: 33, and the light chain variable region CDRL3 consisting of the amino acid sequence as set forth in SEQ ID NO: 34.

The above-described each CDR includes CDR consisting of amino acid sequence comprising a deletion, substitution or addition of one or several, preferably 1 or 2, more preferably 1 amino acid, with respect to each relevant amino acid sequence.

The aforementioned anti-EPHA2 monoclonal antibody comprising the above-described heavy chain variable region and light chain variable region is composed of the above-described heavy chain variable region and a heavy chain constant region and the above-described light chain variable region and a light chain constant region. The heavy chain constant region is composed of three domains C_{H}1, C_{H}2 and C_{H}3. The heavy chain constant region may be an IgG1, IgG2, IgG3, IgG4, IgA, IgE, IgM or IgD constant region. The heavy chain constant region is preferably an IgG1 or IgG2 constant region, and is most preferably an IgG1 constant region. The light chain constant region is composed of a single domain C_{L}. The light chain constant region is a κ or λ constant region.

The antibody of the present invention also includes a functional fragment of the antibody or a modified form thereof. For example, the functional fragment of the antibody is a fragment of the antibody that can specifically bind to an antigen. Examples of the functional fragment include Fab, Fab', F(ab')₂, disulfide-stabilized Fv (dsFv), a dimeric V region (diabody), and single chain Fv (scFv).

The antibody of the present invention can be generated using antibody-producing hybridomas. The antibody of the present invention can also be generated as a recombinant antibody from cells, by inserting DNA encoding a heavy chain variable region or DNA encoding a light chain variable region into an expression vector, then transforming host cells for use in expression with the vector, and then culturing the host cells.

The antibody of the present invention includes an antibody that binds to an epitope, to which the above-described antibody binds, and an antibody that competes against the above-described antibody regarding the binding to EPHA2.

In the present invention, the anti-human EPHA2 monoclonal antibody is used to construct an immunoassay system. Examples of the immunoassay include immunoblotting, enzyme immunoassay (EIA and ELISA), radioimmunoassay (RIA), a fluorescent antibody technique, a method utilizing an agglutination reaction, and immunochromatography.

In addition, in the present invention, the anti-human EPHA2 monoclonal antibody is used to construct a sandwich immunoassay system, among such immunoassay systems. The sandwich immunoassay includes all sandwich immunoassay methods, such as sandwich radioimmunoassay (RIA), sandwich enzyme immunoassay (EIA), sandwich fluorescence immunoassay (FIA), sandwich chemiluminescence immunoassay (CLIA), sandwich chemiluminescent enzyme immunoassay (CLEIA), and immunochromatography based on a sandwich method. Among these methods, ELISA (Enzyme-Linked ImmunoSorbent Assay) that is one of EIA methods is preferable. Hereafter, ELISA will be described. Other sandwich immunoassay methods are the same as ELISA in that an antigen to be measured is sandwiched between two types of antibodies, and thus, a person skilled in the art could construct other sandwich immunoassay measurement systems. An unlabeled anti-human EPHA2 monoclonal antibody is immobilized as a capturing antibody (primary antibody) on a carrier such as a 96-well microtiter plate or polystyrene beads. Moreover, as a detection antibody (secondary antibody), an anti-human EPHA2 monoclonal antibody, which is labeled with an enzyme such as horse radish peroxidase (HRP) or alkaline phosphatase (ALP), a fluorescent substance such as FITC, or biotin, is used. In this case, a Fab' fragment, which is obtained by digesting an antibody molecule with pepsin, may be labeled with an enzyme or the like. Otherwise, after the anti-human EPHA2 monoclonal antibody has been reacted with an unlabeled secondary antibody, it may be then reacted with an antibody specifically binding to the secondary antibody, which is labeled with an enzyme, a fluorescent substance, etc. and is used as a third antibody for use in detection. At this time, the secondary antibody may be labeled with biotin, and the labeled avidin or streptavidin may be then bound to biotin for coloration. The antigen-antibody reaction can be carried out at a temperature of 4°C to 45°C, more preferably 20°C to 40°C, and further preferably 25°C to 38°C. The reaction time is approximately 10 minutes to 18 hours, more preferably approximately 10 minutes to 1 hour, and further preferably approximately 30 minutes to 1 hour. The concentration of EPHA2 in a sample can be calculated from a standard curve, which has previously been prepared regarding the degree of coloration, or which is simultaneously prepared upon the measurement of a sample collected from a subject. In the sandwich immunoassay, either the antibody 57-1 or the antibody 230-1 is immobilized on a carrier, such as a microtiter plate for ELISA, and is used as a solid-phase antibody, whereas the other antibody is labeled and is used as a detection antibody. The antibody 57-1 may be used as a solid-phase antibody, and the antibody 230-1 may be used as a detection antibody. Otherwise, the antibody 57-1 may be used as a detection antibody, and the antibody 230-1 may be used as a solid-phase antibody. A method, in which these monoclonal antibodies are used, has high specificity, detects only EPHA1, and does not show cross-reactivity to EPHA1, EPHA3, and EPHA4.

The present invention includes a test kit for measuring EPHA2, or a composition for use in immunological diagnosis, which comprises at least two types of antibodies, namely, the antibody 57-1 and the antibody 230-1. Either the antibody 57-1 or the antibody 230-1 may be immobilized on a microtiter plat for ELISA, and the other antibody may be labeled. The above-described test kit may include a specimen collecting tool, a specimen treating solution, and a reagent such as a chromogenic substrate, and may further include tools necessary for the test, and the like.

By measuring EPHA2 in the blood of a subject, whether or not the subject suffers from cancer can be determined. That is to say, when the concentration of EPHA2 in the blood of a subject is higher than the concentration of EPHA2 in the blood of a healthy donor, it can be determined that the subject suffers from cancer. Otherwise, the concentration of EPHA2 in the biological sample of a healthy donor has previously been measured, and based on the measured value, a cutoff value (threshold) may be set with regard to the measured value of the concentration of EPHA2. Using the cutoff value as a reference, when the measured value exceeds the cutoff value, it can be determined that the subject suffers from cancer.

The cancer is preferably gastric cancer, ovarian cancer, breast cancer, esophageal cancer, prostate cancer, non-small-cell lung cancer, colon cancer, pancreatic cancer, liver cancer, bladder cancer, cervical cancer, uterine cancer, kidney cancer, brain tumor, malignant melanoma, head and neck cancer, or the like.

The present invention includes a method for detecting cancer using the concentration of EPHA2 in blood as an indicator. This method is also a method for obtaining auxiliary data that are used to detect cancer using the concentration of EPHA2 in blood as an indicator.

The present invention includes a test kit or a composition for use in immunological diagnosis, which comprises at least the antibody 57-1 or the antibody 230-1, and is used to measure EPHA2 and detect cancer. The above-described test kit may include a specimen collecting tool, a specimen treating solution, and a reagent such as a chromogenic substrate, and may further include tools necessary for the test, and the like.

Further, the present invention includes a test kit or a composition for use in immunological diagnosis, which comprises at least two types of antibodies, namely, the antibody 57-1 and the antibody 230-1, and is used to measure EPHA2 and detect cancer. The above-described test kit may include a specimen collecting tool, a specimen treating solution, and a reagent such as a chromogenic substrate, and may further include tools necessary for the test, and the like.

### Examples

Hereinafter, the present invention will be more specifically described in the following examples. However, these examples are not intended to limit the scope of the present invention. It is to be noted that, in the following examples, individual operations regarding genetic operations were carried out according to the methods described in "Molecular Cloning," Sambrook, J., Fritsch, E. F. and Maniatis, T., published by Cold Spring Harbor Laboratory Press in 1989, unless otherwise clearly specified. Alternatively, when commercially available reagents or kits were used, they were used in accordance with the instruction manuals included with the commercially available products.

### [Example 1]

### Production of anti-human EPHA2 monoclonal antibody

### 1)-1 Production of plasmid encoding human EPHA2

### 1)-1-1 Production of plasmid encoding full-length human EPHA

A PCR reaction was carried out using, as a template, cDNA synthesized from the total RNA of SK-OV-3 cells, and also using the following primer set:
Primer 1: 5'-ggggacaagtttgtacaaaaaagcaggcttcggggatcggaccgagagcgagaag-3' (SEQ ID NO: 1 in the sequence listing); and
Primer 2: 5'-ggggaccactttgtacaagaaagctgggtcctagatggggatccccacagtgttcacctggtcctt-3' (SEQ ID NO: 2 in the sequence listing), thereby amplifying cDNA encoding human EPHA2. The PCR fragment was cloned into pDONR221 (Invitrogen, Thermo Fisher Scientific), using BP Clonase (Invitrogen, Thermo Fisher Scientific). From the cloned EPHA2 gene, a stop codon was removed using GeneTailor Site-Directed Mutagenesis System (Invitrogen, Thermo Fisher Scientific) and the following primer set:
Primer 3: 5'-ctgtggggatccccatcgacccagctttc-3' (SEQ ID NO: 3 in the sequence listing); and
Primer 4: 5'-gatggggatccccacagtgttcacctggtc-3' (SEQ ID NO: 4 in the sequence listing).

This EPHA2 gene fragment was transferred into pcDNA-DEST40 Gateway Vector (Invitrogen, Thermo Fisher Scientific), using LR Clonase (Invitrogen, Thermo Fisher Scientific). Hereinafter, this plasmid is referred to as "pcDNA-DEST40-EPHA2" (the present plasmid has the nucleotide sequence as set forth in SEQ ID NO: 5 in the sequence listing between the attB1 and attB2 sequences). Moreover, the coding sequence of the EPHA2 gene cloned into the present plasmid is shown as nucleotide numbers 33 to 2960 of SEQ ID NO: 5 in the sequence listing. Furthermore, the amino acid sequence of EPHA2 is as set forth in SEQ ID NO: 6 in the sequence listing.

### 1)-1-2 Production of plasmid encoding extracellular region of EPHA2

A gene encoding the extracellular region of human EPHA2 (consisting of the amino acid sequence as shown in amino acid numbers 1 to 534 of SEQ ID NO: 6 in the sequence listing; hereinafter referred to as "EPHA2 (ECR)") was amplified by a PCR reaction using the following primer set:
Primer 5: 5'-aaaaagcttatggagctccaggcagcccgc-3' (SEQ ID NO: 7 in the sequence listing); and
Primer 6: 5'-aaagggccctcagttgccagatccctccgg-3' (SEQ ID NO: 8 in the sequence listing). The PCR fragment was cleaved with HindIII and ApaI, and was then cloned into the HindIII/ApaI site of pcDNA3.1 (hereinafter referred to as "pcDNA3.1-EPHA2 (ECR)"; and also, a recombinant protein expressed by pcDNA3.1-EPHA2 (ECR) is referred to as "EPHA2 (ECR)").

### 1)-2 Production of plasmid encoding human ERBB2

A PCR reaction was carried out using Human clone collection (STRATAGENE #C33830, Agilent Technologies) as a template, and also using the following primer set: Primer 7: 5'-caccatggagctggcggccttg-3' (SEQ ID NO: 9 in the sequence listing); and Primer 8: 5'-tcccactggcacgtccagacc-3' (SEQ ID NO: 10 in the sequence listing). The obtained PCR fragment was cloned into pENTR/D-TOPO (Invitrogen, Thermo Fisher Scientific), using pENTR Directional TOPO Cloning kit (Invitrogen, Thermo Fisher Scientific). In order to repair a mutation involving amino acid substitution, among fragments obtained by treating this plasmid with EcoRI, a fragment comprising a sequence derived from pENTR/D-TOPO was ligated to a second largest fragment (approx. 1.6 kbp), among fragments obtained by treating Human clone collection (STRATAGENE #C14640, Agilent Technologies) with EcoRI. An ERBB2 gene fragment in this plasmid was transferred into a pcDNA-DEST40 Gateway vector, using LR Clonase. Hereinafter, this plasmid is referred to as "pcDNA-DEST40-ERBB2" (the present plasmid has the nucleotide sequence as set forth in SEQ ID NO: 11 in the sequence listing between the attB1 and attB2 sequences).

### 1)-3 Preparation of antigen protein

In order to express EPHA2 (ECR), FreeStyle 293-F cells (Invitrogen, Thermo Fisher Scientific) were transfected with pcDNA3.1-EPHA2 (ECR), using 293fectin (Invitrogen, Thermo Fisher Scientific), and the cells were then cultured at 37°C in 8% CO₂ for 5 days. The culture supernatant was recovered by centrifugation, and was then dialyzed against 20 mM Tris-HCl (pH 7.5), using a dialysis tube with a molecular weight fraction of 15000. The culture supernatant was passed through a filter (0.45 µm, PES), and was then loaded on HiPrep 16/10 Q XL (GE Healthcare) equilibrated with 20 mM Tris-HCl (pH 7.5). The binding protein was eluted with a linear concentration gradient of NaCl (20 mM Tris-HCl (pH7.5), 0-1 M NaCl). Subsequently, a fraction comprising EPHA2 (ECR) was collected, and was then loaded on HiLoad 26/60 Superdex 200pg (GE Healthcare) equilibrated with PBS. The protein was eluted with PBS. A fraction comprising EPHA2 (ECR) was collected and was then used as an antigen for immunization. The concentration of the protein was measured using BCA Protein Assay Reagent (PIERCE, Thermo Fisher Scientific).

### 1)-4 Immunization

As initial immunization, 50 µg of EPHA2 (ECR), which had been mixed with Adjuvant Complete Freund H37 Rv (Wako Pure Chemical) or TiterMax Gold Adjuvant (SIGMA), was administered into the dorsal subcutis of BALB/cAnNCrlCrlj mice (Charles River Laboratories Japan). As second and third immunizations, 50 µg of EPHA2 (ECR), which had been mixed with Adjuvant Incomplete Freund (Wako Pure Chemical), was administered into the dorsal subcutis of the mice. These mice were boosted with 50 µg of EPHA2 (ECR), and three days later, the spleen was collected. This spleen was used to produce hybridomas.

### 1)-5 Production of hybridomas

The splenic cells were fused with mouse myeloma P3X63Ag8U.1 cells, using PEG4000 (IBL), so as to produce hybridomas. Using a culture supernatant of the hybridomas, anti-EPHA2 antibody-producing hybridomas were screened.

### 1)-6 Antibody screening

### 1)-6-1 Preparation of antigen-expressing cells

293T cells were seeded at a density of 5 x 10⁴ cells/cm² on a collagen type I-coated flask (IWAKI), and were then cultured in DMEM supplemented with 10% FBS at 37°C in 5% CO₂ under a humidified atmosphere overnight. On the following day, the 293T cells were transfected with each of pcDNA-DEST40-EPHA2 and pcDNA-DEST40-ERBB2 (negative control), using Lipofectamine 2000 (Invitrogen, Thermo Fisher Scientific), and thereafter, the resulting cells were further cultured at 37°C in 5% CO₂ under a humidified atmosphere overnight. On the following day, the transfected cells were treated with trypsin, and were then washed with DMEM supplemented with 10% FBS. The resulting cells were suspended in 5% FBS-containing PBS. These cells were used in Cell-ELISA and flow cytometric analysis.

### 1)-6-2 Cell-ELISA

The cells prepared in the above 1)-6-1 were mixed with the culture supernatant of the hybridomas, and the thus obtained mixture was then incubated at 4°C for 1 hour. The resulting cells were washed with 5% FBS-containing PBS twice, and were then stained with Goat anti-Mouse IgG, Peroxidase Conjugated (Millipore (Chemicon) #AP181P), which had been 500-fold diluted with 5% FBS-containing PBS, at 4°C for 1 hour. Thereafter, the resulting cells were washed with 5% FBS-containing PBS twice, and an OPD coloring solution (o-phenylenediamine dihydrochloride (Wako Pure Chemical Industries, Ltd.) and H₂O₂ were added to an OPD solution (0.05 M trisodium citrate and 0.1 M disodium hydrogen phosphate 12-water (pH 4.5)) to concentrations of 0.4 mg/ml and 0.6% (v/v), respectively) was then added in an amount of 100 µl/well to the resulting cells. While stirring, a coloring reaction was carried out, and 1 M HCl was then added in an amount of 100 µl/well to the reaction mixture to terminate the coloring reaction. After centrifugation, the obtained supernatant was transferred to a novel 96-well flat bottom microplate, and the absorbance at 490 nm was then measured using a plate reader (ARVO: PerkinElmer). In order to select hybridomas generating an antibody specifically binding to EPHA2, hybridomas exhibiting higher absorbance to EPHA2-expressing 293T cells, than to ERBB2-expressing 293T cells (negative control), were selected.

### 1)-6-3 Flow cytometric analysis

In order to eliminate false-positive results in the Cell-ELISA, the specific binding ability of antibodies generated by the hybridomas that had been determined to be positive in the Cell-ELISA was confirmed by flow cytometry. The cells prepared in the above 1)-6-1 were mixed with a culture supernatant of the hybridomas, and the obtained mixture was then incubated at 4°C for 1 hour. The reaction mixture was washed with 5% FBS-containing PBS twice, and was then stained with Fluorescein-conjugated goat IgG fraction to mouse IgG (Whole Molecule) (ICN Pharmaceuticals #55493), which had been 1000-fold diluted with 5% FBS-containing PBS, at 4°C for 1 hour. The resultant was washed with 5% FBS-containing PBS twice, and was then re-suspended in 5% FBS-containing PBS that comprised 2 µg/ml 7-aminoactinomycin D (Invitrogen (Molecular Probes), Thermo Fisher Scientific). The re-suspension was subjected to an analysis using a flow cytometer (FC500: BeckmanCoulter). For data analysis, Flowjo (Tree Star) was used. Dead cells that were exhibited positive to 7-aminoactinomycin D were eliminated with a gate, and thereafter, a histogram of the FITC fluorescence intensity of living cells was prepared. Hybridomas, which generated antibodies exhibiting higher binding ability to EPHA2-expressing 293T cells than to ERBB2-expressing 293T cells used as controls in the histogram, were selected.

### 1)-7 Monocloning of hybridomas

Hybridomas generating anti-EPHA2 antibodies were cultured using ClonaCell-HY Selection Medium D (StemCell Technologies #03804), and appearing colonies were then recovered, so that the monocloned cells were obtained. Using a culture supernatant of the thus monocloned cells, the binding ability of the monocloned cells to EPHA2 was confirmed by the same method as that in the above 1)-6-3. As a result, hybridomas generating the anti-EPHA2 monoclonal antibodies (57-1, 80-21, 83-21, 113-1, 119-1, 131-1, 141-21, 148-1, 151-4, 230-1, 240-4, and 245-21) were established.

### 1)-8 Determination of isotypes of monoclonal antibodies

The isotypes of the monoclonal antibodies were determined using Mouse monoclonal isotyping kit (Serotec). As a result, the isotypes of the monoclonal antibodies were found to be IgG1 (57-1, 113-1, 119-1, 131-1, 141-21, and 230-1), IgG2a (80-21, 83-21, 148-1, 151-4, and 240-4), and IgG2b (245-21).

### [Example 2]

### Development of measurement method of EPHA2

### 2)-1 Selection of monoclonal antibodies for use in ELISA

### 2)-1-1 Coating of anti-EPHA2 monoclonal antibodies

In order to select anti-EPHA2 monoclonal antibodies that can be used in sandwich ELISA for measuring EPHA2, the anti-EPHA2 monoclonal antibodies SHM41 (Toyo University, Non Patent Literatures 19 and 20), 57-1, 80-21, 83-21, 113-1, 119-1, 131-1, 141-21, 148-1, 151-4, 230-1, 240-4, and 245-21 were diluted to a concentration of 5 µg/ml with PBS, immediately before coating, and the thus obtained solutions were each added in an amount of 100 µl/well to an EIA/RIA plate high binding type flat bottom 96-well plate (Costar: No. 9018). A plate seal was attached to the plate, and the antibodies were then adsorbed on the plate at 4°C for 16 hours or more, followed by performing a blocking treatment. For blocking, an assay solution (ASSAY DILUENT: BIOLEGEND) was 5-fold diluted with deionized water and was then used, and the resultant was left at rest at 4°C overnight.

### 2)-1-2 Reference protein

As a human EPHA2 reference protein, human recombinant EphA2-(x6 His) purchased from R & D was used. The following five stages of dilution concentrations were set: 2000 pg/ml, 500 pg/ml, 125 pg/ml, 31.3 pg/ml, and 0 pg/ml. After the removal of the blocking solution from the plate of the above 2)-1-1, each concentration of EPHA2 reference protein solution was added in an amount of 100 µl/well to the plate, and it was then reacted at room temperature for 2 hours.

### 2)-1-3 Biotin-labeled detection antibody

The solution was removed from the plate of the above 2)-1-2 after completion of the reaction, and an ELISA washing solution (0.05% Tween20-containing PBS solution) was then added in an amount of 200 µl/well to the plate. After the obtained mixture had been stirred using a plate mixer (AS ONE Corp.) for 15 seconds, the washing solution was removed. This washing operation was repeated three times, and thereafter, a biotin-labeled anti-EPHA2 polyclonal antibody (R & D: BAF3035) that had been 1000-fold diluted with an assay solution was added in an amount of 100 µl/well to the reaction solution. The obtained mixture was reacted at room temperature for 1 hour.

### 2)-1-4 Avidin enzyme solution

The biotin antibody solution was removed from the plate of the above 2)-1-3 after completion of the reaction, and thereafter, the operation to wash the solution with an ELISA washing solution was repeated four times. Thereafter, Streptavidin-HRP (BD Bioscience) that had been 4000-fold diluted with an assay solution was added in an amount of 100 µl/well to the reaction solution. The obtained mixture was reacted at room temperature for 45 minutes.

### 2)-1-5 Substrate solution

The biotin antibody solution was removed from the plate of the above 2)-1-4 after completion of the reaction, and thereafter, the operation to wash the solution with an ELISA washing solution was repeated five times. Thereafter, TMB (3,3',5,5'-Tetramethylbenzidine) solution (KPL) was added in an amount of 100 µl/well to the reaction solution. The obtained mixture was reacted at room temperature for 15 minutes, so that it was allowed to develop blue color.

### 2)-1-6 Reaction termination

A 2 N (2 normal) sulfuric acid solution (Wako Pure Chemical) was added in an amount of 100 µl/well to the reaction mixture, so that the solution was changed to yellow color as a result of termination of the reaction. Thereafter, the absorbance at 450 nm (reference wavelength: 570 nm) was measured using a microplate reader iMark (BIO-RAD).

### 2)-1-7 Results

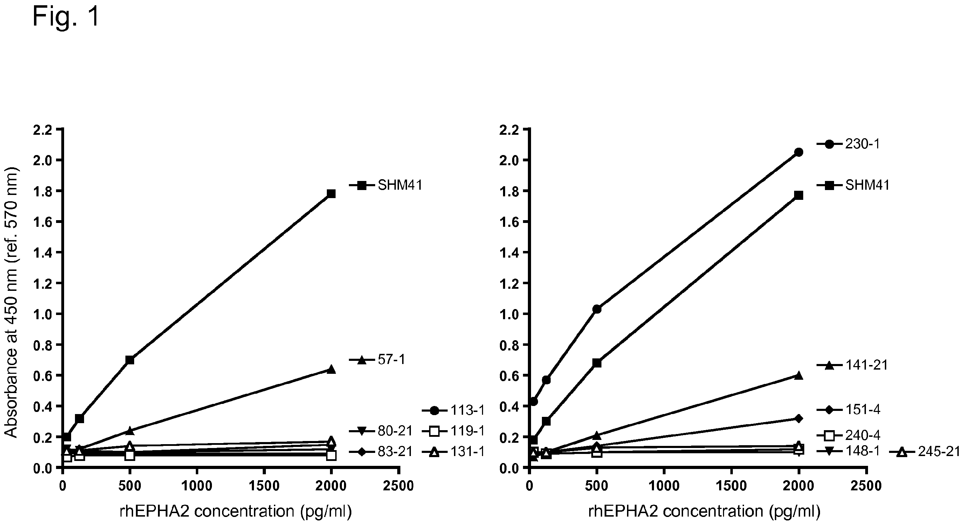

Reactivity was observed in SHM41, 230-1, 57-1, 141-21, and 151-4, and among others, 230-1 exhibited the highest reactivity (Figure 1).

### 2)-2 Sandwich ELISA using anti-EPHA2 monoclonal antibody 230-1

### 2)-2-1 Coating of anti-EPHA2 monoclonal antibodies

In order to select an anti-EPHA2 monoclonal antibody that was to be combined with the anti-EPHA2 monoclonal antibody 230-1, the antibody 230-1 was labeled with biotin, and it was then used as a detection antibody. As capture antibodies, the antibodies 57-1, 80-21, 83-21, 113-1, 119-1, 131-1, 141-21, 148-1, 151-4, 240-4, 245-21 and the isotype IgG1 were each diluted with PBS to a concentration of 5 µg/ml, immediately before coating, and thereafter, the antibodies were each added in an amount of 100 µl/well to an EIA/RIA plate high binding type flat bottom 96-well plate. A plate seal was attached to the plate, and the antibodies were then adsorbed on the plate at 4°C for 16 hours or more, followed by performing a blocking treatment. For blocking, an assay solution was 5-fold diluted with deionized water and was then used, and the resultant was left at rest at 4°C overnight.

### 2)-2-2 Reference protein

The human EPHA2 reference protein was diluted to 4 stages of dilution concentrations, namely, 2000 pg/ml, 500 pg/ml, 125 pg/ml, and 0 pg/ml. After the removal of the blocking solution from the plate of the above 2)-2-1, each concentration of EPHA2 reference protein solution was added in an amount of 100 µl/well to the plate, and it was then reacted at room temperature for 2 hours.

### 2)-2-3 Biotinylation labeling

The monoclonal antibody 230-1 was subjected to biotinylation labeling using a biotin labeling kit (Dojindo Laboratories) in accordance with experimental procedures.

### 2)-2-4 Biotin-labeled detection antibody

The solution was removed from the plate of the above 2)-2-2 after completion of the reaction, and an ELISA washing solution was then added in an amount of 200 µl/well to the plate. After the obtained mixture had been stirred using a plate mixer for 15 seconds, the washing solution was removed. This washing operation was repeated three times, and thereafter, a biotin-labeled anti-EPHA2 monoclonal antibody 230-1 that had been 1000-fold diluted with an assay solution was added in an amount of 100 µl/well to the reaction solution. The obtained mixture was reacted at room temperature for 1 hour.

### 2)-2-5 Avidin enzyme solution

The biotin antibody solution was removed from the plate of the above 2)-2-4 after completion of the reaction, and thereafter, the operation to wash the solution with an ELISA washing solution was repeated four times. Thereafter, Streptavidin-HRP (BIOLEGEND) that had been 2000-fold diluted with an assay solution was added in an amount of 100 µl/well to the reaction solution. The obtained mixture was reacted at room temperature for 45 minutes.

### 2)-2-6 Substrate solution

The biotin antibody solution was removed from the plate of the above 2)-2-5 after completion of the reaction, and thereafter, the operation to wash the solution with an ELISA washing solution was repeated five times. Thereafter, a TMB solution was added in an amount of 100 µl/well to the reaction solution. The obtained mixture was reacted at room temperature for 15 minutes, so that it was allowed to develop blue color.

### 2)-2-7 Reaction termination

A 2 N sulfuric acid solution was added in an amount of 100 µl/well to the reaction mixture, so that the solution was changed to yellow color as a result of termination of the reaction. Thereafter, the absorbance at 450 nm (reference wavelength: 570 nm) was measured using a microplate reader.

### 2)-2-8 Results

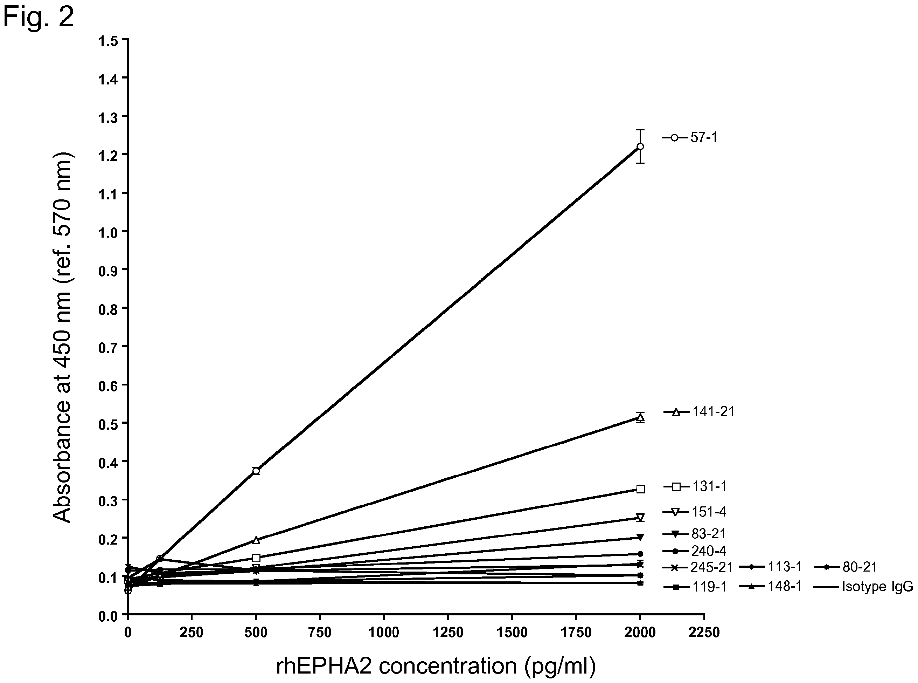

In the case of using the biotin-labeled antibody 230-1, it exhibited the highest reactivity, when it was combined with the antibody 57-1, among the examined 11 types of anti-EPHA2 monoclonal antibodies (Figure 2). 2)-3 Sandwich ELISA using anti-EPHA2 antibodies 230-1, 57-1 and SHM41 2)-3-1 Coating of anti-EPHA2 monoclonal antibodies

As coating antibodies used in sandwich ELISA for detection of EPHA2, the antibodies 57-1, 230-1, and SHM41 were each diluted with PBS to a concentration of 5 µg/ml, and thereafter, the antibodies were each added in an amount of 100 µl/well to an EIA/RIA plate high binding type flat bottom 96-well plate. A plate seal was attached to the plate, and the antibodies were then adsorbed on the plate at 4°C for 16 hours or more, followed by performing a blocking treatment. For blocking, an assay solution was 5-fold diluted with deionized water and was then used, and the resultant was left at rest at 4°C overnight.

### 2)-3-2 Reference protein

A human EPHA2 reference protein was serially (every 2-fold) diluted from a dilution concentration of 2000 pg/ml to 8 stages of concentrations (minimal concentration: 7.8 pg/ml). The blocking solution was removed from the plate of the above 2)-3-1, and each concentration of EPHA2 reference protein solution was added in an amount of 100 µl/well to the plate, and it was then reacted at room temperature for 2 hours.

### 2)-3-3 Biotin-labeled detection antibody

The solution was removed from the plate of the above 2)-3-2 after completion of the reaction, and an ELISA washing solution was then added in an amount of 200 µl/well to the plate. After the obtained mixture had been stirred for 15 seconds using a plate mixer, the washing solution was removed. This washing operation was repeated three times, and thereafter, a biotin-labeled anti-EPHA2 monoclonal antibody (57-1 or 230-1) that had been 1000-fold diluted with an assay solution was added in an amount of 100 µl/well to the reaction solution. The obtained mixture was reacted at room temperature for 1 hour.

### 2)-3-4 Avidin enzyme solution

The biotin antibody solution was removed from the plate of the above 2)-3-3 after completion of the reaction, and thereafter, the operation to wash the solution with an ELISA washing solution was repeated four times. Thereafter, Streptavidin-HRP that had been 2000-fold diluted with an assay solution was added in an amount of 100 µl/well to the reaction solution. The obtained mixture was reacted at room temperature for 45 minutes.

### 2)-3-5 Substrate solution

The biotin antibody solution was removed from the plate of the above 2)-3-4 after completion of the reaction, and thereafter, the operation to wash the solution with an ELISA washing solution was repeated five times. Thereafter, a TMB solution was added in an amount of 100 µl/well to the reaction solution. The obtained mixture was reacted at room temperature for 15 minutes, so that it was allowed to develop blue color.

### 2)-3-6 Reaction termination

A 2 N sulfuric acid solution was added in an amount of 100 µl/well to the reaction mixture, so that the solution was changed to yellow color as a result of termination of the reaction. Thereafter, the absorbance at 450 nm (reference wavelength: 570 nm) was measured using a microplate reader.

### 2)-3-7 Results

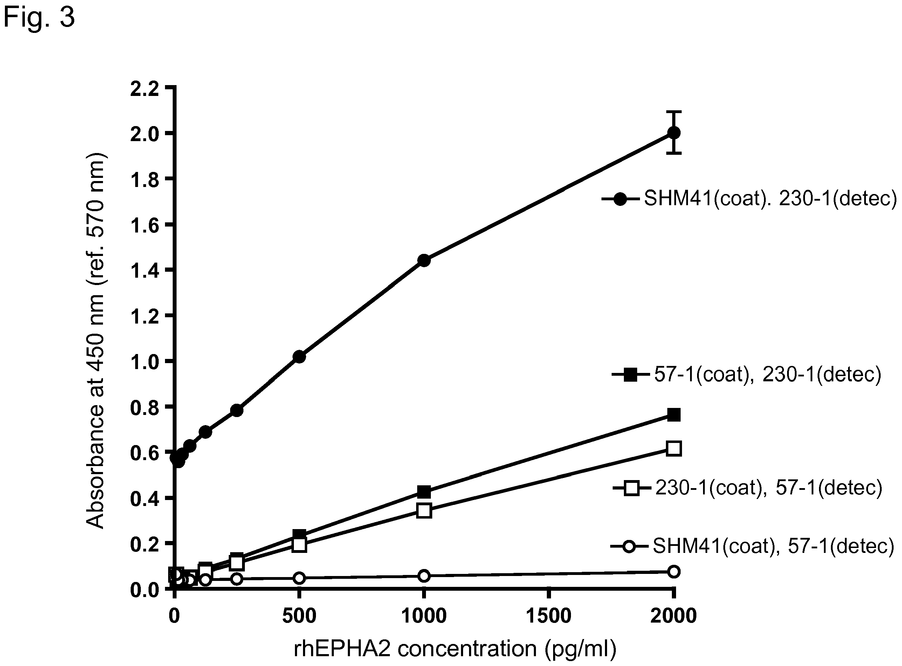

In a system in which SHM41 was used as a capture antibody and the biotin-labeled antibody 57-1 was used as a detection antibody, EPHA2 was hardly detected. In addition, in a system in which SHM41 was used as a capture antibody and the biotin-labeled antibody 230-1 was used as a detection antibody, EPHA2 was detected, but the background was high. On the other hand, in a system in which the antibody 57-1 or 230-1 was used as a capture antibody, and the biotin-labeled antibody 57-1 or the biotin-labeled antibody 230-1 was used as a detection antibody, it was possible to detect EPHA2, and the background was low. From these results, it was demonstrated that the combination of the antibody 57-1 and the antibody 230-1 was promising for detection of EPHA2 (Figure 3).

### 2)-4 Construction of EPHA2 sandwich ELISA system

In order to optimize an ELISA system of using the antibodies 57-1 and 230-1, a blocking solution, a diluted solution of the reference protein, and a diluted solution of the biotin-labeled antibody were changed.

### 2)-4-1 Coating of anti-EPHA2 monoclonal antibody

As coating antibodies used in sandwich ELISA for detection of EPHA2, the antibodies 57-1 and 230-1 were each diluted with PBS to a concentration of 5 µg/ml, and thereafter, the antibodies were each added in an amount of 100 µl/well to an EIA/RIA plate high binding type flat bottom 96-well plate. A plate seal was attached to the plate, and the antibodies were then adsorbed on the plate at 4°C for 16 hours or more, followed by performing a blocking treatment. For blocking, SuperBlock Blocking Solution (THERMO SCIENTIFIC) was used, and the resultant was left at rest at 4°C overnight.

### 2)-4-2 Reference protein

A human EPHA2 reference protein was serially (every 2-fold) diluted from a dilution concentration of 2000 pg/ml to 8 stages of concentrations (minimal concentration: 7.8 pg/ml), using Can Get Signal Solution #1 (TOYOBO LIFE SCIENCE). The blocking solution was removed from the plate of the above 2)-4-1, and each concentration of EPHA2 reference protein solution was added in an amount of 100 µl/well to the plate, and it was then reacted at room temperature for 2 hours.

### 2)-4-3 Biotin-labeled detection antibody

The solution was removed from the plate of the above 2)-4-2 after completion of the reaction, and an ELISA washing solution was then added in an amount of 200 µl/well to the plate. After the obtained mixture had been stirred for 15 seconds using a plate mixer, the washing solution was removed. This washing operation was repeated three times, and thereafter, a biotin-labeled anti-EPHA2 monoclonal antibody (57-1 or 230-1) that had been 1000-fold diluted with Can Get Signal Solution #2 (TOYOBO LIFE SCIENCE) was added in an amount of 100 µl/well to the reaction solution. The obtained mixture was reacted at room temperature for 1 hour.

### 2)-4-4 Avidin enzyme solution

The biotin antibody solution was removed from the plate of the above 2)-4-3 after completion of the reaction, and thereafter, the operation to wash the solution with an ELISA washing solution was repeated four times. Thereafter, Streptavidin-HRP that had been 2000-fold diluted with an assay solution was added in an amount of 100 µl/well to the reaction solution. The obtained mixture was reacted at room temperature for 45 minutes.

### 2)-4-5 Substrate solution

The biotin antibody solution was removed from the plate of the above 2)-4-4 after completion of the reaction, and thereafter, the operation to wash the solution with an ELISA washing solution was repeated five times. Thereafter, a TMB solution was added in an amount of 100 µl/well to the reaction solution. The obtained mixture was reacted at room temperature for 15 minutes, so that it was allowed to develop blue color.

### 2)-4-6 Reaction termination

A 2 N sulfuric acid solution was added in an amount of 100 µl/well to the reaction mixture, so that the solution was changed to yellow color as a result of termination of the reaction. Thereafter, the absorbance at 450 nm (reference wavelength: 570 nm) was measured using a microplate reader.

### 2)-4-7 Results

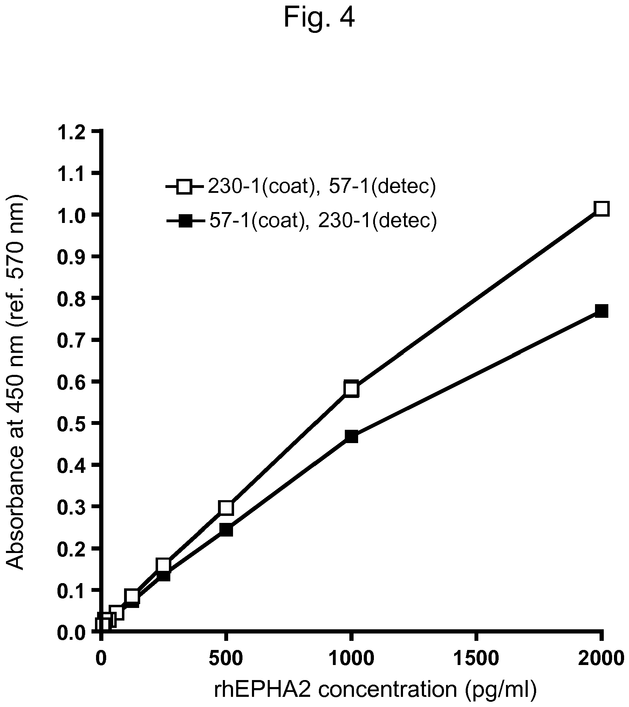

Both in a system in which the antibody 57-1 was used as a capture antibody and the biotin-labeled antibody 230-1 was used as a detection antibody, and in a system in which the antibody 230-1 was used as a capture antibody and the biotin-labeled antibody 57-1 was used as a detection antibody, it was possible for these ELISA systems to detect EPHA2, and the background was low. Moreover, in a system in which the antibody 230-1 was used as a capture antibody and the biotin-labeled antibody 57-1 was used as a detection antibody, a higher absorbance value was exhibited in a high concentration region (Figure 4).

### 2)-5 Calibration curve

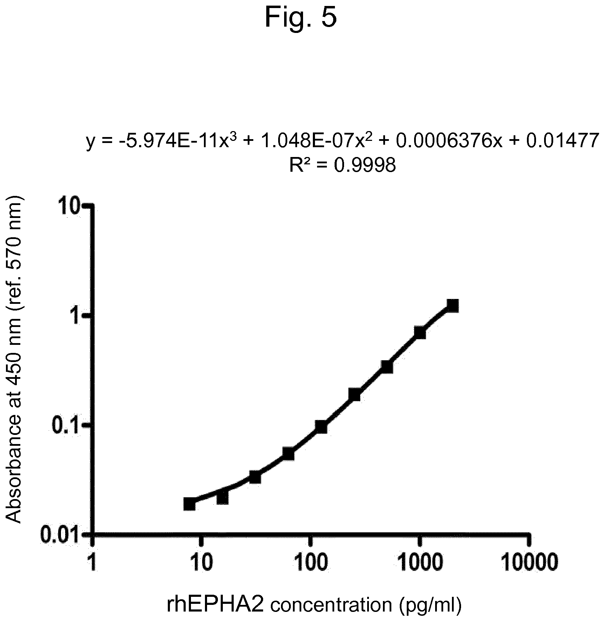

In accordance with the method of Example 2)-4, the antibody 230-1 was used as a coating antibody (capture antibody) in sandwich ELISA, the biotin-labeled antibody 57-1 was used as a detection antibody, and a human EPHA2 reference protein that had been serially diluted from 2000 pg/ml to 7.8 pg/ml was measured. A calibration curve was produced using a cubic polynomial model of Prism 4 (version 4.03, Graph Pad Software, Inc.). As a result, the R² value was 0.9998, and the calibration curve showed favorable compatibility in the range from 7.8 pg/ml to 2000 pg/ml (Figure 5).

### 2)-6 Detection specificity

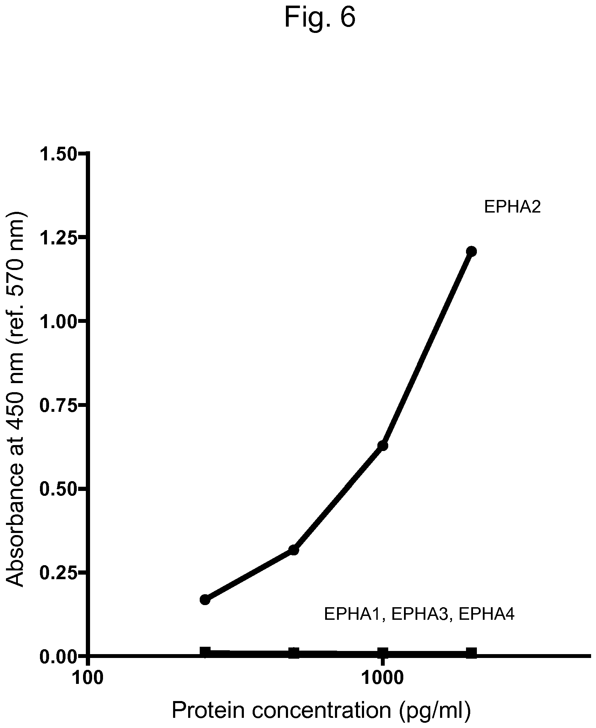

Using the method of Example 2)-4, other than the EPHA2 protein, EPHA1, EPHA3 and EPHA4 proteins (SINO BIOLOGICAL) were each diluted to 2000 pg/ml, 1000 pg/ml, 500 pg/ml, and 250 pg/ml in the same manner as described above, and were then measured according to sandwich ELISA. As a result, only EPHA2 was detected by this method, and no cross-reactivity was shown with EPHA1, EPHA3, and EPHA4 (Figure 6). From these results, it was demonstrated that this ELISA system has high specificity to EPHA2.

### 2)-7 Measurement of EPHA2 value in human serum

### 2)-7-1 Serum derived from cancer patients

Cryopreserved serums derived from gastric cancer patients (10 cases), cryopreserved serums derived from ovarian cancer patients (10 cases) and cryopreserved serums derived from healthy donors (10 cases) were acquired from BIOSERVE (agency: ReproCELL). All of the above-described serums were attended with IRB certificates proving the consent of the donors.

Cryopreserved serums derived from non-small-cell lung cancer patients (10 cases) and cryopreserved serums derived from healthy donors (10 cases) were acquired from ProteoGenex (agency: BizCom Japan, Inc.). All of the above-described serums were attended with IRB certificates proving the consent of the donors.

### 2)-7-2 Measurement of EPHA2 value in serum

In accordance with the method of Example 2)-4, various types of serums (from healthy donors, gastric cancer patients, and ovarian cancer patients) were 5-fold diluted with Can Get Signal Solution #1, and were then measured by sandwich ELISA.

### 2)-7-3 statistical analysis

A statistical analysis was carried out using Steel test (SAS System Release 9.2, SAS Institute Inc.).

### 2)-7-4 Results

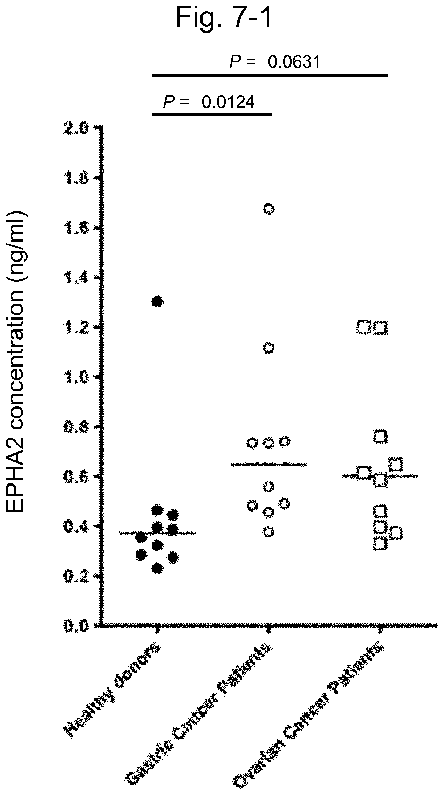

The medians of the EPHA2 concentrations in the serums of healthy donors, gastric cancer patients, and ovarian cancer patients were 372 pg/ml, 647 pg/ml, and 600 pg/ml, respectively. The EPHA2 concentration in the blood of the gastric cancer patients was significantly higher than that in the healthy donors (P = 0.0142) (Figure 7-1).

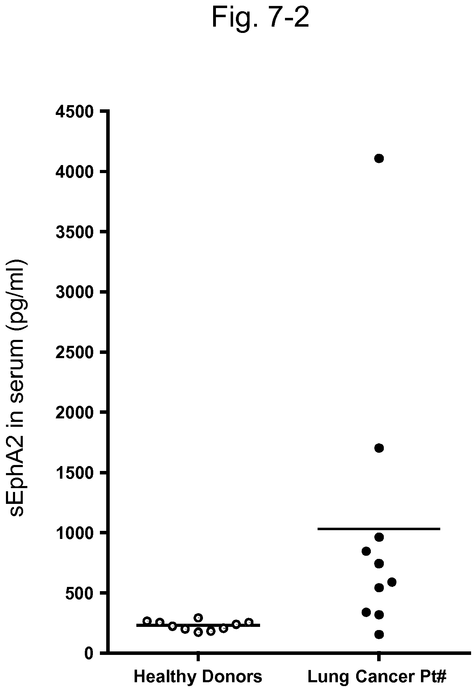

The medians of the EPHA2 concentrations in the serums of healthy donors and non-small-cell lung cancer patients were 232 pg/ml and 645 pg/ml, respectively. In addition, the mean values thereof were 231 pg/ml and 1,031 pg/ml, respectively. The EPHA2 concentration in the blood of the non-small-cell lung cancer patients was significantly higher than that in the healthy donors (P = 0.0024) (Figure 7-2).

### [Example 3]

### Determination of nucleotide sequence of cDNA encoding variable region of mouse anti-EPHA2 monoclonal antibody

### 3)-1 Determination of nucleotide sequence of cDNA encoding variable region of mouse anti-EPHA2 antibody 57-1

### 3)-1-1 Preparation of total RNA from antibody 57-1 producing hybridomas

Total RNA was prepared from the antibody 57-1 producing hybridomas, using TRIzol Reagent (Ambion).

### 3)-1-2 Synthesis of cDNA (5'-RACE-Ready cDNA)

Using 1 µg of the total RNA prepared in Example 3)-1-1 and SMARTer RACE 5'/3' Kit (Clontech), cDNA (5'-RACE-Ready cDNA) was synthesized.

### 3)-1-3 Amplification of cDNA encoding heavy chain variable region of antibody 57-1 according to 5'-RACE PCR, and determination of nucleotide sequence thereof

In order to amplify cDNA encoding the heavy chain variable region of the antibody 57-1 according to PCR, the following primers were used:
UPM (Universal Primer A Mix: included with SMARTer RACE 5'/3' Kit), and
5'-CATCCCAGGGTCACCATGGAGTTAGTTTGG-3' (mG1VR1: SEQ ID NO: 12). The mG1VR1 was designed based on the sequence of the constant region of the mouse heavy chain (IgG1) shown in database.

Using these primers, and also using the cDNA (5'-RACE-Ready cDNA) synthesized in Example 3)-1-2 as a template, cDNA encoding the heavy chain variable region of the antibody 57-1 was amplified according to 5'-RACE PCR. This PCR was carried out using KOD-Plus- (TOYOBO) as polymerase, by the touchdown PCR program described in the instruction manual of SMARTer RACE 5'/3' Kit.

The amplified cDNA fragment was purified using MinElute PCR Purification Kit (QIAGEN), and was then cloned using Zero Blunt TOPO PCR Cloning Kit (Invitrogen, Thermo Fisher Scientific).

In order to determine the nucleotide sequence of the cloned cDNA fragment, the following sequencing primers were used:
5'-CATCCCAGGGTCACCATGGAGTTAGTTTGG-3' (mG1VR1: SEQ ID NO: 12); and
5'-AAGCAGTGGTATCAACGCAGAGT-3' (NUP: SEQ ID NO: 13).

The sequencing reaction was carried out using GeneAmp 9700 (Applied Biosystems, current Thermo Fisher Scientific), and the sequencing of the DNA was carried out using ABI PRISM 3700 DNA Analyzer (Applied Biosystems, current Thermo Fisher Scientific) or Applied Biosystems 3730xl Analyzer (Applied Biosystems, current Thermo Fisher Scientific).

The determined nucleotide sequence of the cDNA encoding the heavy chain variable region of the antibody 57-1 is as set forth in SEQ ID NO: 14 (Figure 8), and the amino acid sequence thereof is as set forth in SEQ ID NO: 15 (Figure 9).

### 3)-1-4 Amplification of cDNA encoding light chain variable region of antibody 57-1 according to 5'-RACE PCR, and determination of nucleotide sequence thereof

In order to amplify cDNA encoding the light chain variable region of the antibody 57-1 according to PCR, the following primers were used:
UPM (Universal Primer A Mix: included with SMARTer RACE 5'/3' Kit), and
5'-AGTCCAACTGTTCAGGACGCCATTTTGTCG-3'(mKVR2:SEQ ID NO: 16).
The mKVR2 was designed based on the sequence of the constant region of the mouse light chain shown in database.

Using these primers, and also using the cDNA (5'-RACE-Ready cDNA) synthesized in Example 3)-1-2 as a template, cDNA encoding the light chain variable region of the antibody 57-1 was amplified according to 5'-RACE PCR. This PCR was carried out using KOD-Plus- (TOYOBO) as polymerase, by the touchdown PCR program described in the instruction manual of SMARTer RACE 5'/3' Kit.

The amplified cDNA fragment was purified using MinElute PCR Purification Kit (QIAGEN), and was then cloned using Zero Blunt TOPO PCR Cloning Kit (Invitrogen, Thermo Fisher Scientific).

In order to determine the nucleotide sequence of the cloned cDNA fragment, the following sequencing primers were used:
5'-AGTCCAACTGTTCAGGACGCCATTTTGTCG-3' (mKVR2: SEQ ID NO: 16); and
5'-AAGCAGTGGTATCAACGCAGAGT-3' (NUP: SEQ ID NO: 13).

The sequencing reaction was carried out using GeneAmp 9700 (Applied Biosystems, current Thermo Fisher Scientific), and the sequencing of the DNA was carried out using ABI PRISM 3700 DNA Analyzer (Applied Biosystems, current Thermo Fisher Scientific) or Applied Biosystems 3730xl Analyzer (Applied Biosystems, current Thermo Fisher Scientific).

The determined nucleotide sequence of the cDNA encoding the light chain variable region of the antibody 57-1 is as set forth in SEQ ID NO: 17 in the sequence listing (Figure 10), and the amino acid sequence thereof is as set forth in SEQ ID NO: 18 (Figure 11).

### 3)-2 Determination of nucleotide sequence of cDNA encoding variable region of mouse anti-EPHA2 antibody 230-1

### 3)-2-1 Preparation of total RNA from antibody 230-1 producing hybridomas

Total RNA was prepared from the antibody 230-1 producing hybridomas by the same method as that of Example 3)-1-1.

### 3)-2-2 Synthesis of cDNA (5'-RACE-Ready cDNA)

Using 1 µg of the total RNA prepared in Example 3)-2-1, cDNA (5'-RACE-Ready cDNA) was synthesized by the same method as that of Example 3)-1-2.

### 3)-2-3 Amplification of cDNA encoding heavy chain variable region of antibody 230-1 according to 5'-RACE PCR, and determination of nucleotide sequence thereof

Using the cDNA (5'-RACE-Ready cDNA) synthesized in Example 3)-2-2 as a template, cDNA encoding the heavy chain variable region of the antibody 230-1 was amplified by the same method as that of Example 3)-1-3, and the nucleotide sequence thereof was then determined.

The determined nucleotide sequence of the cDNA encoding the heavy chain variable region of the antibody 230-1 is as set forth in SEQ ID NO: 19 in the sequence listing (Figure 12), and the amino acid sequence thereof is as set forth in SEQ ID NO: 20 (Figure 13).

### 3)-2-4 Amplification of cDNA encoding light chain variable region of antibody 230-1 according to 5'-RACE PCR, and determination of nucleotide sequence thereof

Using the cDNA (5'-RACE-Ready cDNA) synthesized in Example 3)-2-2 as a template, cDNA encoding the light chain variable region of the antibody 230-1 was amplified by the same method as that of Example 3)-1-4, and the nucleotide sequence thereof was then determined.

The determined nucleotide sequence of the cDNA encoding the light chain variable region of the antibody 230-1 is as set forth in SEQ ID NO: 21 in the sequence listing (Figure 14), and the amino acid sequence thereof is as set forth in SEQ ID NO: 22 (Figure 15).

### Industrial Applicability

The antibody of the present invention is suitable for measuring EPHA2 in a biological sample by a sandwich immunoassay, and can be utilized in detection of cancer, etc.

### Sequence Listing Free Text

SEQ ID NOS: 1 to 4, 7 to 10, 12, 13 and 16 Primers

All publications, patents and patent applications cited in the present description are incorporated herein by reference in their entirety.

## Claims

1. A monoclonal antibody specifically binding to EPHA2, comprising:
(a) a heavy chain CDRH1 consisting of the amino acid sequence as set forth in SEQ ID NO: 29,
(b) a heavy chain CDRH2 consisting of the amino acid sequence as set forth in SEQ ID NO: 30,
(c) a heavy chain CDRH3 consisting of the amino acid sequence as set forth in SEQ ID NO: 31,
(d) a light chain CDRL1 consisting of the amino acid sequence as set forth in SEQ ID NO: 32,
(e) a light chain CDRL2 consisting of the amino acid sequence as set forth in SEQ ID NO: 33, and
(f) a light chain CDRL3 consisting of the amino acid sequence as set forth in SEQ ID NO: 34, or
a functional fragment of the monoclonal antibody specifically binding to EPHA2.

2. A monoclonal antibody specifically binding to EPHA2, comprising a heavy chain variable region consisting of the amino acid sequence as set forth in SEQ ID NO: 20 and a light chain variable region consisting of the amino acid sequence as set forth in SEQ ID NO: 22, or a functional fragment of the monoclonal antibody specifically binding to EPHA2.

3. A monoclonal antibody specifically binding to EPHA2, comprising: a heavy chain variable region consisting of an amino acid sequence having a sequence identity of 95% or more to the amino acid sequence as set forth in SEQ ID NO: 20, and having binding activity to human EPHA2; and a light chain variable region consisting of an amino acid sequence having a sequence identity of 95% or more to the amino acid sequence as set forth in SEQ ID NO: 22, and having binding activity to human EPHA2, or a functional fragment of the monoclonal antibody specifically binding to EPHA2.

4. A monoclonal antibody specifically binding to EPHA2, comprising:
(a) a heavy chain CDRH1 consisting of the amino acid sequence as set forth in SEQ ID NO: 23,
(b) a heavy chain CDRH2 consisting of the amino acid sequence as set forth in SEQ ID NO: 24,
(c) a heavy chain CDRH3 consisting of the amino acid sequence as set forth in SEQ ID NO: 25,
(d) a light chain CDRL1 consisting of the amino acid sequence as set forth in SEQ ID NO: 26,
(e) a light chain CDRL2 consisting of the amino acid sequence as set forth in SEQ ID NO: 27, and
(f) a light chain CDRL3 consisting of the amino acid sequence as set forth in SEQ ID NO: 28, or
a functional fragment of the monoclonal antibody specifically binding to EPHA2.

5. A monoclonal antibody specifically binding to EPHA2, comprising a heavy chain variable region consisting of the amino acid sequence as set forth in SEQ ID NO: 15 and a light chain variable region consisting of the amino acid sequence as set forth in SEQ ID NO: 18, or a functional fragment of the monoclonal antibody specifically binding to EPHA2.

6. A monoclonal antibody specifically binding to EPHA2, comprising: a heavy chain variable region consisting of an amino acid sequence having a sequence identity of 95% or more to the amino acid sequence as set forth in SEQ ID NO: 15, and having binding activity to human EPHA2; and a light chain variable region consisting of an amino acid sequence having a sequence identity of 95% or more to the amino acid sequence as set forth in SEQ ID NO: 18, and having binding activity to human EPHA2, or a functional fragment of the monoclonal antibody specifically binding to EPHA2.

7. A combination of two types of monoclonal antibodies; namely, the monoclonal antibody specifically binding to EPHA2 according to any one of claims 1 to 3, or a functional fragment of the monoclonal antibody specifically binding to EPHA2, and the monoclonal antibody specifically binding to EPHA2, or a functional fragment of the monoclonal antibody specifically binding to EPHA2 according to any one of claims 4 to 6.

8. The combination according to claim 7, which is used in a method for detecting or measuring EPHA2 in a biological sample.

9. A method for specifically detecting EPHA2 contained in a biological sample according to an immunoassay, which uses the monoclonal antibody specifically binding to EPHA2, or a functional fragment of the monoclonal antibody specifically binding to EPHA2 according to any one of claims 1 to 6.

10. A method for specifically detecting EPHA2 contained in a biological sample according to a sandwich immunoassay, which uses two types of monoclonal antibodies; namely, the monoclonal antibody specifically binding to EPHA2, or a functional fragment of the monoclonal antibody specifically binding to EPHA2 according to any one of claims 1 to 3, and the monoclonal antibody specifically binding to EPHA2, or a functional fragment of the monoclonal antibody specifically binding to EPHA2 according to any one of claims 4 to 6.

11. The method according to claim 10, wherein the sandwich immunoassay is ELISA.

12. A method for detecting cancer, which comprises: measuring the concentration of EPHA2 in the blood of a subject according to an immunoassay, using the monoclonal antibody specifically binding to EPHA2, or a functional fragment of the monoclonal antibody specifically binding to EPHA2 according to any one of claims 1 to 6; and evaluating that the subject suffers from cancer, when the concentration of EPHA2 in the blood of the subject is higher than the concentration of EPHA2 in the blood of a healthy donor.

13. A method for detecting cancer, which comprises: measuring the concentration of EPHA2 in the blood of a subject according to a sandwich immunoassay, using two types of monoclonal antibodies, namely, the monoclonal antibody specifically binding to EPHA2, or a functional fragment of the monoclonal antibody specifically binding to EPHA2 according to any one of claims 1 to 3, and the monoclonal antibody specifically binding to EPHA2, or a functional fragment of the monoclonal antibody specifically binding to EPHA2 according to any one of claims 4 to 6; and evaluating that the subject suffers from cancer, when the concentration of EPHA2 in the blood of the subject is higher than the concentration of EPHA2 in the blood of a healthy donor.

14. The method according to claim 13, wherein the sandwich immunoassay is ELISA.

15. The method according to any one of claims 12 to 14, wherein the cancer is selected from the group consisting of gastric cancer, ovarian cancer, breast cancer, esophageal cancer, prostate cancer, non-small-cell lung cancer, colon cancer, pancreatic cancer, liver cancer, bladder cancer, cervical cancer, uterine cancer, kidney cancer, brain tumor, malignant melanoma, and head and neck cancer.

16. A kit for measuring EPHA2, comprising two types of monoclonal antibodies or functional fragments thereof; namely, the monoclonal antibody specifically binding to EPHA2, or a functional fragment of the monoclonal antibody specifically binding to EPHA2 according to any one of claims 1 to 3, and the monoclonal antibody specifically binding to EPHA2, or a functional fragment of the monoclonal antibody specifically binding to EPHA2 according to any one of claims 4 to 6.

17. A kit for detecting cancer, comprising two types of monoclonal antibodies or functional fragments thereof; namely, the monoclonal antibody specifically binding to EPHA2, or a functional fragment of the monoclonal antibody specifically binding to EPHA2 according to any one of claims 1 to 3, and the monoclonal antibody specifically binding to EPHA2, or a functional fragment of the monoclonal antibody specifically binding to EPHA2 according to any one of claims 4 to 6.

18. The kit according to claim 16, wherein the cancer is selected from the group consisting of gastric cancer, ovarian cancer, breast cancer, esophageal cancer, prostate cancer, non-small-cell lung cancer, colon cancer, pancreatic cancer, liver cancer, bladder cancer, cervical cancer, uterine cancer, kidney cancer, brain tumor, malignant melanoma, and head and neck cancer.
